# EUROPEAN PATENT SPECIFICATION

(11) **EP 1 320 593 B1**
(45) Date of publication and mention of the grant of the patent: **23.04.2008**
(21) Application number: 01978296.0
(22) Date of filing: 23.08.2001
(51) Int. Cl.: C12N 15/00

(54) **Process for the preparation of L-lysine using CYSD, CYSN, CYSK, CYSE AND/or CYSH OF C. GLUTAMICUM**
Verfahren zur Herstellung von L-Lysine unter Verwendung von CYSD, CYSN, CYSK, CYSE und/oder CYSH VON C. GLUTAMICUM
Procédé de préparation de la L-lysine en utilisant CYSD, CYSN, CYSK, CYSE ET/OU CYSH de C. Glutamicum

(30) Priority: 30.09.2000 DE 10048603; 28.02.2001 DE 10109691; 28.07.2001 DE 10136986
(43) Date of publication of application: 25.06.2003
(73) Proprietor: Evonik Degussa GmbH, 40474 Düsseldorf (DE)
(72) Inventor: FARWICK, Mike, 33615 Bielefeld (DE); HUTHMACHER, Klaus, 63571 Gelnhausen (DE); SCHISCHKA, Natalie, 33659 Bielefeld (DE); BATHE, Brigitte, 33154 Salzkotten (DE); PFEFFERLE, Walter, 33790 Halle (Westf.) (DE); BINDER, Michael, 33803 Steinhagen (Westf.) (DE); GREISSINGER, Dieter, 61194 Niddatal (DE); THIERBACH, Georg, 33613 Bielefeld (DE)
(86) International application number: PCT/EP2001/009723
(87) International publication number: WO 2002/029029

(56) References cited:
- EP-A- 1 006 192
- EP-A- 1 108 790
- WO-A-01/00842
- WO-A-01/00843
- WO-A-97/15673
- DATABASE EBI [Online] EMBL; MMCV_STRLA, 30 May 2000 (2000-05-30) XP002191266

### SEQUENCE PROTOCOL

<110> Degussa AG
<120> Nucleotide sequences which code for the cysD, cysN, cysK, cysE and cysH genes
<130> 000491 BT
<140>
   <141>
<160> 16
<170> Patent In Ver. 2.1
<210> 1
   <211> 2640
   <212> DNA
   <213> Corynebacterium glutamicum
<220>
   <221> CDS
   <222> (232)..(1143)
   <223> cysD gene
<220>
   <221> CDS
   <222> (1146)..(2444)
   <223> cysN gene
<400> 1
   tgcgctgagc ttggatgcca ccggcaggct caagatttct ccaattatca cctggtcatt 60
   ggaggaaacc aacgagttca ttgcggacaa caacctcatc gatcacccac ttacccatca 120
   gggttatcca tcaattggat gcgaaacctg cacccttcct gttgctgaag gacaagaccc 180
   tagggccggc cgttgggctg gaaacgccaa gacagaatgc ggacttcact c atg acc  237
                                                           Met Thr
                                                             1
   aca acc gtt gca tca gta cta tcc cca cac ctt aaa gat ctt gaa aat   285
   Thr Thr Val Ala Ser Val Leu Ser Pro His Leu Lys Asp Leu Glu Asn
            5                  10                  15
   gaa tcc atc cac atc ctc cgc gag gta gct ggc cag ttt gat aag gtc   333
   Glu Ser Ile His Ile Leu Arg Glu Val Ala Gly Gln Phe Asp Lys Val
        20                  25                  30
   ggc ctg ctg ttt tcc ggc ggt aag gat tcc gtc gtg gtg tac gag ctt   381
   Gly Leu Leu Phe Ser Gly Gly Lys Asp Ser Val Val Val Tyr Glu Leu
    35                  40                  45                  50
   gcg cgc cgc gct ttc gct cca gct aac gtg cct ttt gaa ttg ctg cac   429
   Ala Arg Arg Ala Phe Ala Pro Ala Asn Val Pro Phe Glu Leu Leu His
                    55                  60                  65
   gtg gac acc ggc cac aac ttc cca gag gtt ttg gaa ttc cgc gac aac      477
   Val Asp Thr Gly His Asn Phe Pro Glu Val Leu Glu Phe Arg Asp Asn
               70                  75                  80
   ctg gtg gag cgc acc ggc gcc cgc ctg cgc gta gct aaa gtc cag gac      525
   Leu Val Glu Arg Thr Gly Ala Arg Leu Arg Val Ala Lys Val Gln Asp
           85                  90                  95
   tgg atc gat cgc ggt gac ctg cag gaa cgc cca gac ggc acc cgc aac      573
   Trp Ile Asp Arg Gly Asp Leu Gln Glu Arg Pro Asp Gly Thr Arg Asn
       100                 105                 110
   cca ctg cag act gtc cct ttg gtg gag acc atc gct gag cag ggc tac      621
   Pro Leu Gln Thr Val Pro Leu Val Glu Thr Ile Ala Glu Gln Gly Tyr
   115                 120                 125                 130
   gac gca gtg ctt ggt ggc gct cgc cgc gat gag gag cgt gcc cgc gcc      669
   Asp Ala Val Leu Gly Gly Ala Arg Arg Asp Glu Glu Arg Ala Arg Ala
                   135                 140                 145
   aag gag cgt gtg ttc tct gtg cgt gac tcc ttc ggt ggt tgg gat cca      717
   Lys Glu Arg Val Phe Ser Val Arg Asp Ser Phe Gly Gly Trp Asp Pro
               150                 155                 160
   cgc cgt cag cgc cca gag ctg tgg acc ctc tac aac ggt ggc cac ctg      765
   Arg Arg Gln Arg Pro Glu Leu Trp Thr Leu Tyr Asn Gly Gly His Leu
           165                 170                 175
   cca ggc gaa aac atc cgt gtt ttc cca atc tcc aac tgg act gaa gct      813
   Pro Gly Glu Asn Ile Arg Val Phe Pro Ile Ser Asn Trp Thr Glu Ala
       180                 185                 190
   gac att tgg gag tac atc ggc gcc cgt ggc atc gaa ctt cca ccg atc      861
   Asp Ile Trp Glu Tyr Ile Gly Ala Arg Gly Ile Glu Leu Pro Pro Ile
   195                 200                 205                 210
   tac ttc tcc cac gac cgc gaa gtt ttc gag cgc gac ggc atg tgg ctg      909
   Tyr Phe Ser His Asp Arg Glu Val Phe Glu Arg Asp Gly Met Trp Leu
                   215                 220                 225
   acc gca ggc gag tgg ggt gga cca aag aag ggc gag gag atc gtc acc      957
   Thr Ala Gly Glu Trp Gly Gly Pro Lys Lys Gly Glu Glu Ile Val Thr
               230                 235                 240
   aag act gtc cgc tac cgc acc gtc ggc gat atg tcc tgc acc ggt gct      1005
   Lys Thr Val Arg Tyr Arg Thr Val Gly Asp Met Ser Cys Thr Gly Ala
           245                 250                 255
   gtg ctc tcc gaa gcc cgc acc att gac gat gtg atc gaa gag atc gcc      1053
   Val Leu Ser Glu Ala Arg Thr Ile Asp Asp Val Ile Glu Glu Ile Ala
       260                 265                 270
   acc tcc acc ctt acc gaa cgt ggc gca acc cgc gcc gat gac cgc ctc      1101
   Thr Ser Thr Leu Thr Glu Arg Gly Ala Thr Arg Ala Asp Asp Arg Leu
   275                 280                 285                 290
   agc gaa tcc gca atg gaa gac cgc aag aag gaa ggc tac ttc tg atg      1148
   Ser Glu Ser Ala Met Glu Asp Arg Lys Lys Glu Gly Tyr Phe    Met
                   295                 300                 305
   act gct cca acc ttg aat aaa gca tcc gaa aag att gca tca cgc gag    1196
   Thr Ala Pro Thr Leu Asn Lys Ala Ser Glu Lys Ile Ala Ser Arg Glu
                   310                 315                 320
   acc ctt cgt ctg tgc acc gca ggt tcc gta gat gat ggc aag tcc acc    1244
   Thr Leu Arg Leu Cys Thr Ala Gly Ser Val Asp Asp Gly Lys Ser Thr
               325                 330                 335
   ttc gtc ggc cgc ctc ctg cac gac acc aag tct gtt ctt gct gat cag    1292
   Phe Val Gly Arg Leu Leu His Asp Thr Lys Ser Val Leu Ala Asp Gln
           340                 345                 350
   ctg gct tcc gta gag cgc acc tcc gcc gac cgt ggc ttc gaa ggc ctc    1340
   Leu Ala Ser Val Glu Arg Thr Ser Ala Asp Arg Gly Phe Glu Gly Leu
       355                 360                 365
   gac ctg tca ctc ctc gtc gac ggc ctg cgc gcc gag cgt gag cag ggc    1388
   Asp Leu Ser Leu Leu Val Asp Gly Leu Arg Ala Glu Arg Glu Gln Gly
   370                 375                 380                 385
   atc acc atc gac gtt gcc tac cgc tac ttc gcc acc gac aag cgc acc    1436
   Ile Thr Ile Asp Val Ala Tyr Arg Tyr Phe Ala Thr Asp Lys Arg Thr
                   390                 395                 400
   ttc atc ctg gct gat acc cca ggt cac gtg cag tac acc cgc aac acc    1484
   Phe Ile Leu Ala Asp Thr Pro Gly His Val Gln Tyr Thr Arg Asn Thr
               405                 410                 415
   gtc acc ggc gtc tcc acc tcc cag gtt gta gtt ttg ctt gtc gac gcc    1532
   Val Thr Gly Val Ser Thr Ser Gln Val Val Val Leu Leu Val Asp Ala
           420                 425                 430
   cgc cac ggc gtc gtc gag cag acc cgc cgc cac ctg tcc gta tcg gct    1580
   Arg His Gly Val Val Glu Gln Thr Arg Arg His Leu Ser Val Ser Ala
       435                 440                 445
   ctg ctg ggc gta cgc acg gtg atc ctc gca gtc aac aaa att gac ctt    1628
   Leu Leu Gly Val Arg Thr Val Ile Leu Ala Val Asn Lys Ile Asp Leu
   450                 455                 460                 465
   gtt gat tac agc gaa gaa gtc ttc cgc aac att gaa aag gaa ttc gtt    1676
   Val Asp Tyr Ser Glu Glu Val Phe Arg Asn Ile Glu Lys Glu Phe Val
                   470                 475                 480
   ggc ctg gca tct gca ctt gat gtc aca gac acc cac gtt gtt cca atc    1724
   Gly Leu Ala Ser Ala Leu Asp Val Thr Asp Thr His Val Val Pro Ile
               485                 490                 495
   tct gcg ctc aag ggc gac aac gtt gca gaa cct tcc acc cac atg gat    1772
   Ser Ala Leu Lys Gly Asp Asn Val Ala Glu Pro Ser Thr His Met Asp
           500                 505                 510
   tgg tac acc gga cca acc gtg ctg gaa atc ctg gaa aac gta gaa gtt    1820
   Trp Tyr Thr Gly Pro Thr Val Leu Glu Ile Leu Glu Asn Val Glu Val
       515                 520                 525
   tcc cac ggc cgt gca cac gac ctg ggc ttc cgc ttc cca atc cag tac    1868
   Ser His Gly Arg Ala His Asp Leu Gly Phe Arg Phe Pro Ile Gln Tyr
   530                 535                 540                 545
   gtc atc cgc gag cac gcc acc gac tac cgt ggc tac gcc ggc acc atc    1916
   Val Ile Arg Glu His Ala Thr Asp Tyr Arg Gly Tyr Ala Gly Thr Ile
                   550                 555                 560
   aac gct ggt tcc gtc tcc gtg ggc gat acc gtg tac cta cct gaa ggc    1964
   Asn Ala Gly Ser Val Ser Val Gly Asp Thr Val Tyr Leu Pro Glu Gly
               565                 570                 575
   cgc acc acc cag gtc acc cac atc gat tcc gct gac gga tcc ctc cag    2012
   Arg Thr Thr Gln Val Thr His Ile Asp Ser Ala Asp Gly Ser Leu Gln
           580                 585                 590
   acc gca tca gtt gga gaa gcc gtt gtc ctg cgc cta gcc cag gaa atc    2060
   Thr Ala Ser Val Gly Glu Ala Val Val Leu Arg Leu Ala Gln Glu Ile
       595                 600                 605
   gac ctc atc cgc ggc gaa ctc atc gct ggc gaa gac cgc cca gaa tcc    2108
   Asp Leu Ile Arg Gly Glu Leu Ile Ala Gly Glu Asp Arg Pro Glu Ser
   610                 615                 620                 625
   gtt cgc tcc ttc aac gcc act gtt gtt ggc ttg gcc gat cgc acc atc    2156
   Val Arg Ser Phe Asn Ala Thr Val Val Gly Leu Ala Asp Arg Thr Ile
                   630                 635                 640
   aaa cca ggt gca gca gtc aag gtt cgc tac ggc acc gag ctg gtc cgc    2204
   Lys Pro Gly Ala Ala Val Lys Val Arg Tyr Gly Thr Glu Leu Val Arg
               645                 650                 655
   gga cgc gtc gca gcc atc gaa cga gtc ctc gac atc gac ggc gtc aac    2252
   Gly Arg Val Ala Ala Ile Glu Arg Val Leu Asp Ile Asp Gly Val Asn
           660                 665                 670
   gac aac gaa gca cca gaa acc tac ggc ctc aac gac atc gca cac gtg    2300
   Asp Asn Glu Ala Pro Glu Thr Tyr Gly Leu Asn Asp Ile Ala His Val
       675                 680                 685
   cgc atc gac gtt gca ggc gaa ctc gaa gtt gaa gat tac gct gcc cgc    2348
   Arg Ile Asp Val Ala Gly Glu Leu Glu Val Glu Asp Tyr Ala Ala Arg
   690                 695                 700                 705
   ggc gcc atc gga tcc ttc ctc ctc atc gac caa tcc tcc ggc gat acc    2396
   Gly Ala Ile Gly Ser Phe Leu Leu Ile Asp Gln Ser Ser Gly Asp Thr
                   710                 715                 720
   ctc gca gct ggc ttg gtt ggc cac cgc cta cgc aat aac tgg tcg atc    2444
   Leu Ala Ala Gly Leu Val Gly His Arg Leu Arg Asn Asn Trp Ser Ile
               725                 730                 735
   tagaccagtg tcttaggcaa gaccccattt aggacacctc atgattcccc tgattacgct 2504
   ttcccacggt tcccgcaaaa agtccgcagc tgcaggcatt actgcgctga ctcatgaggc 2564
   cggacgaatg ctggaaacac cagccgtgga agcgcattta gagcttgctg aaccttccct 2624
   tgatcaggtt gtggca                                                 2640
<210> 2
   <211> 304
   <212> PRT
   <213> Corynebacterium glutamicum
<400> 2
   Met Thr Thr Thr Val Ala Ser Val Leu Ser Pro His Leu Lys Asp Leu
    1               5                  10                  15
   Glu Asn Glu Ser Ile His Ile Leu Arg Glu Val Ala Gly Gln Phe Asp
               20                  25                  30
   Lys Val Gly Leu Leu Phe Ser Gly Gly Lys Asp Ser Val Val Val Tyr
           35                  40                  45
   Glu Leu Ala Arg Arg Ala Phe Ala Pro Ala Asn Val Pro Phe Glu Leu
       50                  55                  60
   Leu His Val Asp Thr Gly His Asn Phe Pro Glu Val Leu Glu Phe Arg
   65                  70                  75                  80
   Asp Asn Leu Val Glu Arg Thr Gly Ala Arg Leu Arg Val Ala Lys Val
                   85                  90                  95
   Gln Asp Trp Ile Asp Arg Gly Asp Leu Gln Glu Arg Pro Asp Gly Thr
               100                 105                 110
   Arg Asn Pro Leu Gln Thr Val Pro Leu Val Glu Thr Ile Ala Glu Gln
           115                 120                 125
   Gly Tyr Asp Ala Val Leu Gly Gly Ala Arg Arg Asp Glu Glu Arg Ala
       130                 135                 140
   Arg Ala Lys Glu Arg Val Phe Ser Val Arg Asp Ser Phe Gly Gly Trp
   145                 150                 155                 160
   Asp Pro Arg Arg Gln Arg Pro Glu Leu Trp Thr Leu Tyr Asn Gly Gly
                   165                 170                 175
   His Leu Pro Gly Glu Asn Ile Arg Val Phe Pro Ile Ser Asn Trp Thr
               180                 185                 190
   Glu Ala Asp Ile Trp Glu Tyr Ile Gly Ala Arg Gly Ile Glu Leu Pro
           195                 200                 205
   Pro Ile Tyr Phe Ser His Asp Arg Glu Val Phe Glu Arg Asp Gly Met
       210                 215                 220
   Trp Leu Thr Ala Gly Glu Trp Gly Gly Pro Lys Lys Gly Glu Glu Ile
   225                 230                 235                 240
   Val Thr Lys Thr Val Arg Tyr Arg Thr Val Gly Asp Met Ser Cys Thr
                   245                 250                 255
   Gly Ala Val Leu Ser Glu Ala Arg Thr Ile Asp Asp Val Ile Glu Glu
               260                 265                 270
   Ile Ala Thr Ser Thr Leu Thr Glu Arg Gly Ala Thr Arg Ala Asp Asp
           275                 280                 285
   Arg Leu Ser Glu Ser Ala Met Glu Asp Arg Lys Lys Glu Gly Tyr Phe
       290                 295                 300
<210> 3
   <211> 433
   <212> PRT
   <213> Corynebacterium glutamicum
<400> 3
   Met Thr Ala Pro Thr Leu Asn Lys Ala Ser Glu Lys Ile Ala Ser Arg
   1               5                   10                  15
   Glu Thr Leu Arg Leu Cys Thr Ala Gly Ser Val Asp Asp Gly Lys Ser
               20                  25                  30
   Thr Phe Val Gly Arg Leu Leu His Asp Thr Lys Ser Val Leu Ala Asp
               35                  40                  45
   Gln Leu Ala Ser Val Glu Arg Thr Ser Ala Asp Arg Gly Phe Glu Gly
           50                  55                  60
   Leu Asp Leu Ser Leu Leu Val Asp Gly Leu Arg Ala Glu Arg Glu Gln
   65                  70                  75                  80
   Gly Ile Thr Ile Asp Val Ala Tyr Arg Tyr Phe Ala Thr Asp Lys Arg
                   85                  90                  95
   Thr Phe Ile Leu Ala Asp Thr Pro Gly His Val Gln Tyr Thr Arg Asn
               100                 105                 110
   Thr Val Thr Gly Val Ser Thr Ser Gln Val Val Val Leu Leu Val Asp
           115                 120                 125
   Ala Arg His Gly Val Val Glu Gln Thr Arg Arg His Leu Ser Val Ser
       130                 135                 140
   Ala Leu Leu Gly Val Arg Thr Val Ile Leu Ala Val Asn Lys Ile Asp
   145                 150                 155                 160
   Leu Val Asp Tyr Ser Glu Glu Val Phe Arg Asn Ile Glu Lys Glu Phe
                   165                 170                 175
   Val Gly Leu Ala Ser Ala Leu Asp Val Thr Asp Thr His Val Val Pro
               180                 185                 190
   Ile Ser Ala Leu Lys Gly Asp Asn Val Ala Glu Pro Ser Thr His Met
           195                 200                 205
   Asp Trp Tyr Thr Gly Pro Thr Val Leu Glu Ile Leu Glu Asn Val Glu
       210                 215                 220
   Val Ser His Gly Arg Ala His Asp Leu Gly Phe Arg Phe Pro Ile Gln
   225                 230                 235                 240
   Tyr Val Ile Arg Glu His Ala Thr Asp Tyr Arg Gly Tyr Ala Gly Thr
                   245                 250                 255
   Ile Asn Ala Gly Ser Val Ser Val Gly Asp Thr Val Tyr Leu Pro Glu
               260                 265                 270
   Gly Arg Thr Thr Gln Val Thr His Ile Asp Ser Ala Asp Gly Ser Leu
           275                 280                 285
   Gln Thr Ala Ser Val Gly Glu Ala Val Val Leu Arg Leu Ala Gln Glu
       290                 295                 300
   Ile Asp Leu Ile Arg Gly Glu Leu Ile Ala Gly Glu Asp Arg Pro Glu
   305                 310                 315                 320
   Ser Val Arg Ser Phe Asn Ala Thr Val Val Gly Leu Ala Asp Arg Thr
                   325                 330                 335
   Ile Lys Pro Gly Ala Ala Val Lys Val Arg Tyr Gly Thr Glu Leu Val
               340                 345                 350
   Arg Gly Arg Val Ala Ala Ile Glu Arg Val Leu Asp Ile Asp Gly Val
           355                 360                 365
   Asn Asp Asn Glu Ala Pro Glu Thr Tyr Gly Leu Asn Asp Ile Ala His
       370                 375                 380
   Val Arg Ile Asp Val Ala Gly Glu Leu Glu Val Glu Asp Tyr Ala Ala
   385                 390                 395                 400
   Arg Gly Ala Ile Gly Ser Phe Leu Leu Ile Asp Gln Ser Ser Gly Asp
                   405                 410                 415
   Thr Leu Ala Ala Gly Leu Val Gly His Arg Leu Arg Asn Asn Trp Ser
               420                 425                 430
   Ile
<210> 4
   <211> 2170
   <212> DNA
   <213> Corynebacterium glutamicum
<220>
   <221> CDS
   <222> (271)..(1203)
   <223> cysK gene
<220>
   <221> CDS
   <222> (1392)..(1955)
   <223> cysE gene
<400> 4
   tccgacaacg gacttcttta aaagatgctt ttcgacgccg ctccccaacc attaaccccg 60
   cgagaaatat tcatcgaata gatgtcgatc tacctgcaaa tacgctcggt ctacaaataa 120
   tgaacagaac tgtctacttt tcaaactgct ttttgtgtag actcaagtca cagaggccac 180
   ttcaagtaga tgtttcgtaa ttgtttacag cgtttacgca agcggtcgac caacaaaaac 240
   agcacttcaa tgattggagc accacccgac atg ggc aat gtg tac aac aac atc  294
                                     Met Gly Asn Val Tyr Asn Asn Ile
                                     1               5
   acc gaa acc atc ggc cac acc cca ctg gta aag ctg aac aag ctc acc   342
   Thr Glu Thr Ile Gly His Thr Pro Leu Val Lys Leu Asn Lys Leu Thr
       10                  15                  20
   gaa ggc ctc gac gca act gtc ctg gtc aag ctt gag tca ttc aac cca   390
   Glu Gly Leu Asp Ala Thr Val Leu Val Lys Leu Glu Ser Phe Asn Pro
   25                  30                  35                  40
   gca aac tcc gtc aag gac cgt atc ggt ctg gcc atc gtt gaa gat gca   438
   Ala Asn Ser Val Lys Asp Arg Ile Gly Leu Ala Ile Val Glu Asp Ala
                   45                  50                  55
   gag aag tcc ggt gca ctg aag cca ggc ggc acc atc gtt gaa gca acc   486
   Glu Lys Ser Gly Ala Leu Lys Pro Gly Gly Thr Ile Val Glu Ala Thr
               60                  65                  70
   tcc ggc aac acc ggt atc gca ctg gca atg gtc ggc gct gca cgc gga   534
   Ser Gly Asn Thr Gly Ile Ala Leu Ala Met Val Gly Ala Ala Arg Gly
           75                  80                  85
   tac aac gtt gtt ctc acc atg ccg gag acc atg tcc aac gag cgt cgc   582
   Tyr Asn Val Val Leu Thr Met Pro Glu Thr Met Ser Asn Glu Arg Arg
       90                  95                  100
   gtt ctc ctc cgc gct tac ggt gca gag atc gtt ctt acc cca ggt gca   630
   Val Leu Leu Arg Ala Tyr Gly Ala Glu Ile Val Leu Thr Pro Gly Ala
   105                 110                 115                 120
   gca ggc atg cag ggt gca aag gac aag gca gac gaa atc gtc gct gaa   678
   Ala Gly Met Gln Gly Ala Lys Asp Lys Ala Asp Glu Ile Val Ala Glu
                   125                 130                 135
   cgc gaa aac gca gtc ctt gct cgc cag ttc gag aac gag gca aac cca   726
   Arg Glu Asn Ala Val Leu Ala Arg Gln Phe Glu Asn Glu Ala Asn Pro
               140                 145                 150
   cgc gtc cac cgc gac acc acc gcg aag gaa atc ctc gaa gac acc gac    774
   Arg Val His Arg Asp Thr Thr Ala Lys Glu Ile Leu Glu Asp Thr Asp
           155                 160                 165
   ggc aac gtt gat atc ttc gtt gca agc ttc ggc acc ggc gga acc gtc    822
   Gly Asn Val Asp Ile Phe Val Ala Ser Phe Gly Thr Gly Gly Thr Val
       170                 175                 180
   acc ggc gtt ggc cag gtc ctg aag gaa aac aac gca gac gta cag gtc    870
   Thr Gly Val Gly Gln Val Leu Lys Glu Asn Asn Ala Asp Val Gln Val
   185                 190                 195                 200
   tac acc gtc gag cca gaa gcg tcc cca ctt ctg acc gct ggc aag gct    918
   Tyr Thr Val Glu Pro Glu Ala Ser Pro Leu Leu Thr Ala Gly Lys Ala
                   205                 210                 215
   ggt cca cac aag atc cag ggc atc ggc gca aac ttc atc ccc gag gtc    966
   Gly Pro His Lys Ile Gln Gly Ile Gly Ala Asn Phe Ile Pro Glu Val
               220                 225                 230
   ctg gac cgc aag gtt ctc gac gac gtg ctg acc gtc tcc aac gaa gac    1014
   Leu Asp Arg Lys Val Leu Asp Asp Val Leu Thr Val Ser Asn Glu Asp
           235                 240                 245
   gca atc gca ttc tcc cgc aag ctc gct acc gaa gag ggc atc ctc ggc    1062
   Ala Ile Ala Phe Ser Arg Lys Leu Ala Thr Glu Glu Gly Ile Leu Gly
       250                 255                 260
   ggt atc tcc acc ggc gca aac atc aag gca gct ctt gac ctt gca gca    1110
   Gly Ile Ser Thr Gly Ala Asn Ile Lys Ala Ala Leu Asp Leu Ala Ala
   265                 270                 275                 280
   aag cca gag aac gct ggc aaa acc atc gtc acc gtt gtc acc gac ttc    1158
   Lys Pro Glu Asn Ala Gly Lys Thr Ile Val Thr Val Val Thr Asp Phe
                   285                 290                 295
   ggc gag cgc tac gtc tcc acc gtt ctt tac gaa gac atc cgc gac        1203
   Gly Glu Arg Tyr Val Ser Thr Val Leu Tyr Glu Asp Ile Arg Asp
               300                 305                 310
   taattcttag cgactgttaa ccactcaagc tctttgcttg ggtggttttt tcatgtctca 1263
   aggtcgggtc gggtgcgatt cgggtcggtt ttgagtgtct ttgagtcctt ttaagtcctt 1323
   ctttgcccgt gaataattct ctggatagtt tccacgtgca gttaagtcac gctgttagac 1383
   ttgcctgc atg ctc tcg aca ata aaa atg atc cgt gaa gat ctc gca aac   1433
            Met Leu Ser Thr Ile Lys Met Ile Arg Glu Asp Leu Ala Asn
                        315                 320                 325
   gct cgt gaa cac gat cca gca gcc cga ggc gat tta gaa aac gca gtg    1481
   Ala Arg Glu His Asp Pro Ala Ala Arg Gly Asp Leu Glu Asn Ala Val
                   330                 335                 340
   gtt tac tcc gga ctc cac gcc atc tgg gca cat cga gtt gcc aac agc    1529
   Val Tyr Ser Gly Leu His Ala Ile Trp Ala His Arg Val Ala Asn Ser
               345                 350                 355
   tgg tgg aaa tcc ggt ttc cgc ggc ccc gcc cgc gta tta gcc caa ttc   1577
   Trp Trp Lys Ser Gly Phe Arg Gly Pro Ala Arg Val Leu Ala Gln Phe
           360                 365                 370
   acc cga ttc ctc acc ggc att gaa att cac ccc ggt gcc acc att ggt   1625
   Thr Arg Phe Leu Thr Gly Ile Glu Ile His Pro Gly Ala Thr Ile Gly
       375                 380                 385
   cgt cgc ttt ttt att gac cac gga atg gga atc gtc atc ggc gaa acc   1673
   Arg Arg Phe Phe Ile Asp His Gly Met Gly Ile Val Ile Gly Glu Thr
   390                 395                 400                     405
   gct gaa atc ggc gaa ggc gtc atg ctc tac cac ggc gtc acc ctc ggc   1721
   Ala Glu Ile Gly Glu Gly Val Met Leu Tyr His Gly Val Thr Leu Gly
                   410                 415                 420
   gga cag gtt ctc acc caa acc aag cgc cac ccc acg ctc tgc gac aac   1769
   Gly Gln Val Leu Thr Gln Thr Lys Arg His Pro Thr Leu Cys Asp Asn
               425                 430                 435
   gtg aca gtc ggc gcg ggc gca aaa atc tta ggt ccc atc acc atc ggc   1817
   Val Thr Val Gly Ala Gly Ala Lys Ile Leu Gly Pro Ile Thr Ile Gly
           440                 445                 450
   gaa ggc tcc gca att ggc gcc aat gca gtt gtc acc aaa gac gtg ccg   1865
   Glu Gly Ser Ala Ile Gly Ala Asn Ala Val Val Thr Lys Asp Val Pro
       455                 460                 465
   gca gaa cac atc gca gtc gga att cct gcg gta gca cgc cca cgt ggc   1913
   Ala Glu His Ile Ala Val Gly Ile Pro Ala Val Ala Arg Pro Arg Gly
   470                 475                 480                     485
   aag aca gag aag atc aag ctc gtc gat ccg gac tat tac att           1955
   Lys Thr Glu Lys Ile Lys Leu Val Asp Pro Asp Tyr Tyr Ile
                   490                 495
   taagaacagt tagcgcccta cctgaagttc aggcagggcg cttttttggg aagctccaga 2015
   gtgcgtttgt tagccacgca ctagggacct ttaaccgtct aaaaccgccc ctgtgcgctt 2075
   ctcagcacta cccgtgagaa ccacccccct gtgccagcta gttctttaga tccttatact 2135
   cagggttctt ctgaatgaag ccagcgactg cagag                            2170
<210> 5
   <211> 311
   <212> PRT
   <213> Corynebacterium glutamicum
<400> 5
   Met Gly Asn Val Tyr Asn Asn Ile Thr Glu Thr Ile Gly His Thr Pro
    1               5                  10                  15
   Leu Val Lys Leu Asn Lys Leu Thr Glu Gly Leu Asp Ala Thr Val Leu
               20                  25                  30
   Val Lys Leu Glu Ser Phe Asn Pro Ala Asn Ser Val Lys Asp Arg Ile
           35                  40                  45
   Gly Leu Ala Ile Val Glu Asp Ala Glu Lys Ser Gly Ala Leu Lys Pro
       50                  55                  60
   Gly Gly Thr Ile Val Glu Ala Thr Ser Gly Asn Thr Gly Ile Ala Leu
   65                  70                  75                  80
   Ala Met Val Gly Ala Ala Arg Gly Tyr Asn Val Val Leu Thr Met Pro
                   85                  90                  95
   Glu Thr Met Ser Asn Glu Arg Arg Val Leu Leu Arg Ala Tyr Gly Ala
               100                 105                 110
   Glu Ile Val Leu Thr Pro Gly Ala Ala Gly Met Gln Gly Ala Lys Asp
           115                 120                 125
   Lys Ala Asp Glu Ile Val Ala Glu Arg Glu Asn Ala Val Leu Ala Arg
       130                 135                 140
   Gln Phe Glu Asn Glu Ala Asn Pro Arg Val His Arg Asp Thr Thr Ala
   145                 150                 155                 160
   Lys Glu Ile Leu Glu Asp Thr Asp Gly Asn Val Asp Ile Phe Val Ala
                   165                 170                 175
   Ser Phe Gly Thr Gly Gly Thr Val Thr Gly Val Gly Gln Val Leu Lys
               180                 185                 190
   Glu Asn Asn Ala Asp Val Gln Val Tyr Thr Val Glu Pro Glu Ala Ser
           195                 200                 205
   Pro Leu Leu Thr Ala Gly Lys Ala Gly Pro His Lys Ile Gln Gly Ile
       210                 215                 220
   Gly Ala Asn Phe Ile Pro Glu Val Leu Asp Arg Lys Val Leu Asp Asp
   225                 230                 235                 240
   Val Leu Thr Val Ser Asn Glu Asp Ala Ile Ala Phe Ser Arg Lys Leu
                   245                 250                 255
   Ala Thr Glu Glu Gly Ile Leu Gly Gly Ile Ser Thr Gly Ala Asn Ile
               260                 265                 270
   Lys Ala Ala Leu Asp Leu Ala Ala Lys Pro Glu Asn Ala Gly Lys Thr
           275                 280                 285
   Ile Val Thr Val Val Thr Asp Phe Gly Glu Arg Tyr Val Ser Thr Val
       290                 295                 300
   Leu Tyr Glu Asp Ile Arg Asp
   305                 310
<210> 6
   <211> 188
   <212> PRT
   <213> Corynebacterium glutamicum
<400> 6
   Met Leu Ser Thr Ile Lys Met Ile Arg Glu Asp Leu Ala Asn Ala Arg
   1               5                   10                  15
   Glu His Asp Pro Ala Ala Arg Gly Asp Leu Glu Asn Ala Val Val Tyr
               20                  25                  30
   Ser Gly Leu His Ala Ile Trp Ala His Arg Val Ala Asn Ser Trp Trp
           35                  40                  45
   Lys Ser Gly Phe Arg Gly Pro Ala Arg Val Leu Ala Gln Phe Thr Arg
       50                  55                  60
   Phe Leu Thr Gly Ile Glu Ile His Pro Gly Ala Thr Ile Gly Arg Arg
   65                  70                  75                  80
   Phe Phe Ile Asp His Gly Met Gly Ile Val Ile Gly Glu Thr Ala Glu
                   85                  90                  95
   Ile Gly Glu Gly Val Met Leu Tyr His Gly Val Thr Leu Gly Gly Gln
               100                 105                 110
   Val Leu Thr Gln Thr Lys Arg His Pro Thr Leu Cys Asp Asn Val Thr
           115                 120                 125
   Val Gly Ala Gly Ala Lys Ile Leu Gly Pro Ile Thr Ile Gly Glu Gly
       130                 135                 140
   Ser Ala Ile Gly Ala Asn Ala Val Val Thr Lys Asp Val Pro Ala Glu
   145                 150                 155                 160
   His Ile Ala Val Gly Ile Pro Ala Val Ala Arg Pro Arg Gly Lys Thr
                   165                 170                 175
   Glu Lys Ile Lys Leu Val Asp Pro Asp Tyr Tyr Ile
               180                 185
<210> 7
   <211> 1240
   <212> DNA
   <213> Corynebacterium glutamicum
<220>
   <221> CDS
   <222> (250)..(1032)
   <223> cysH gene
<400> 7
   tggtgtgagt cttcggagga aacccaatcc caaccgcaac caccctctgt actgcccata 60
   ctgcgcggga gaagttcttt tccccgatga gcaaacagaa ttcgcgtggt tgtgtgcgga 120
   ttgcaccaga gtttttgaag tgaaatatca cggccaggac gatccagtgc acaggccagc 180
   accagcaaag tccacatcgc aagcattaaa agaatctctc gaaagacaca aaagaggtga 240
   gtcgcaaca atg agc ttt caa cta gtt aac gcc ctg aaa aat act ggt tcg 291
             Met Ser Phe Gln Leu Val Asn Ala Leu Lys Asn Thr Gly Ser
             1               5                   10
   gta aaa gat ccc gag atc tca ccc gaa gga cct cgc acg acc aca ccg   339
   Val Lys Asp Pro Glu Ile Ser Pro Glu Gly Pro Arg Thr Thr Thr Pro
   15                  20                  25                  30
   ttg tca cca gag gta gca aaa cat aac gag gaa ctc gtc gaa aag cat   387
   Leu Ser Pro Glu Val Ala Lys His Asn Glu Glu Leu Val Glu Lys His
                   35                  40                  45
   gct gct gcg ttg tat gac gcc agc gcg caa gag atc ctg gaa tgg aca   435
   Ala Ala Ala Leu Tyr Asp Ala Ser Ala Gln Glu Ile Leu Glu Trp Thr
               50                  55                  60
   gcc gag cac gcg ccg ggc gct att gca gtg acc ttg agc atg gaa aac   483
   Ala Glu His Ala Pro Gly Ala Ile Ala Val Thr Leu Ser Met Glu Asn
           65                  70                  75
   acc gtg ctg gcg gag ctg gct gcg cgg cac ctg ccg gaa gct gat ttc   531
   Thr Val Leu Ala Glu Leu Ala Ala Arg His Leu Pro Glu Ala Asp Phe
       80                  85                  90
   ctc ttt ttg gac acc ggt tac cac ttc aag gag acc ctt gaa gtt gcc   579
   Leu Phe Leu Asp Thr Gly Tyr His Phe Lys Glu Thr Leu Glu Val Ala
   95                  100                 105                 110
   cgt cag gta gat gag cgc tat tcc cag aag ctt gtc acc gcg ctg ccg   627
   Arg Gln Val Asp Glu Arg Tyr Ser Gln Lys Leu Val Thr Ala Leu Pro
                   115                 120                 125
   atc ctc aag cgc acg gag cag gat tcc att tat ggt ctc aac ctg tac    675
   Ile Leu Lys Arg Thr Glu Gln Asp Ser Ile Tyr Gly Leu Asn Leu Tyr
               130                 135                 140
   cgc agc aac cca gcg gcg tgc tgc cga atg cgc aaa gtt gaa ccg ctg    723
   Arg Ser Asn Pro Ala Ala Cys Cys Arg Met Arg Lys Val Glu Pro Leu
           145                 150                 155
   gcg gcg tcg tta agc cca tac gct ggc tgg atc acc ggc ctg cgc cgc    771
   Ala Ala Ser Leu Ser Pro Tyr Ala Gly Trp Ile Thr Gly Leu Arg Arg
       160                 165                 170
   gct gat ggc cca acc cgt gct caa gcc cct gcg ctg agc ttg gat gcc    819
   Ala Asp Gly Pro Thr Arg Ala Gln Ala Pro Ala Leu Ser Leu Asp Ala
   175                 180                 185                 190
   acc ggc agg ctc aag att tct cca att atc acc tgg tca ttg gag gaa    867
   Thr Gly Arg Leu Lys Ile Ser Pro Ile Ile Thr Trp Ser Leu Glu Glu
                   195                 200                 205
   acc aac gag ttc att gcg gac aac aac ctc atc gat cac cca ctt acc    915
   Thr Asn Glu Phe Ile Ala Asp Asn Asn Leu Ile Asp His Pro Leu Thr
               210                 215                 220
   cat cag ggt tat cca tca att gga tgc gaa acc tgc acc ctt cct gtt    963
   His Gln Gly Tyr Pro Ser Ile Gly Cys Glu Thr Cys Thr Leu Pro Val
           225                 230                 235
   gct gaa gga caa gac cct agg gcc ggc cgt tgg gct gga aac gcc aag    1011
   Ala Glu Gly Gln Asp Pro Arg Ala Gly Arg Trp Ala Gly Asn Ala Lys
       240                 245                 250
   aca gaa tgc gga ctt cac tca tgaccacaac cgttgcatca gtactatccc      1062
   Thr Glu Cys Gly Leu His Ser
   255                 260
   cacaccttaa agatcttgaa aatgaatcca tccacatcct ccgcgaggta gctggccagt 1122
   ttgataaggt cggcctgctg ttttccggcg gtaaggattc cgtcgtggtg tacgagcttg 1182
   cgcgccgcgc tttcgctcca gctaacgtgc cttttgaatt gctgcacgtg gacaccgg   1240
<210> 8
   <211> 261
   <212> PRT
   <213> Corynebacterium glutamicum
<400> 8
   Met Ser Phe Gln Leu Val Asn Ala Leu Lys Asn Thr Gly Ser Val Lys
   1               5                   10                  15
   Asp Pro Glu Ile Ser Pro Glu Gly Pro Arg Thr Thr Thr Pro Leu Ser
               20                  25                  30
   Pro Glu Val Ala Lys His Asn Glu Glu Leu Val Glu Lys His Ala Ala
           35                  40                  45
   Ala Leu Tyr Asp Ala Ser Ala Gln Glu Ile Leu Glu Trp Thr Ala Glu
       50                  55                  60
   His Ala Pro Gly Ala Ile Ala Val Thr Leu Ser Met Glu Asn Thr Val
   65                  70                  75                  80
   Leu Ala Glu Leu Ala Ala Arg His Leu Pro Glu Ala Asp Phe Leu Phe
                   85                  90                  95
   Leu Asp Thr Gly Tyr His Phe Lys Glu Thr Leu Glu Val Ala Arg Gln
               100                 105                 110
   Val Asp Glu Arg Tyr Ser Gln Lys Leu Val Thr Ala Leu Pro Ile Leu
           115                 120                 125
   Lys Arg Thr Glu Gln Asp Ser Ile Tyr Gly Leu Asn Leu Tyr Arg Ser
       130                 135                 140
   Asn Pro Ala Ala Cys Cys Arg Met Arg Lys Val Glu Pro Leu Ala Ala
   145                 150                 155                 160
   Ser Leu Ser Pro Tyr Ala Gly Trp Ile Thr Gly Leu Arg Arg Ala Asp
                   165                 170                 175
   Gly Pro Thr Arg Ala Gln Ala Pro Ala Leu Ser Leu Asp Ala Thr Gly
               180                 185                 190
   Arg Leu Lys Ile Ser Pro Ile Ile Thr Trp Ser Leu Glu Glu Thr Asn
           195                 200                 205
   Glu Phe Ile Ala Asp Asn Asn Leu Ile Asp His Pro Leu Thr His Gln
       210                 215                 220
   Gly Tyr Pro Ser Ile Gly Cys Glu Thr Cys Thr Leu Pro Val Ala Glu
   225                 230                 235                 240
   Gly Gln Asp Pro Arg Ala Gly Arg Trp Ala Gly Asn Ala Lys Thr Glu
                   245                 250                 255
   Cys Gly Leu His Ser
               260
<210> 9
   <211> 28
   <212> DNA
   <213> Artificial sequence
<220>
   <223> Description of the artificial sequence: Primer cysDex1
<400> 9
   ctggtaccgc ggacttcact catgacca 28
<210> 10
   <211> 28
   <212> DNA
   <213> Artificial sequence
<220>
   <223> Description of the artificial sequence: Primer cysDex2
<400> 10
   cgtctagagg aacctgcggt gcacagac 28
<210> 11
   <211> 28
   <212> DNA
   <213> Artificial sequence
<220>
   <223> Description of the artificial sequence: Primer cysEex1
<400> 11
   ctggtacctc acgctgttag acttgcct 28
<210> 12
   <211> 28
   <212> DNA
   <213> Artificial sequence
<220>
   <223> Description of the artificial sequence: Primer cysEex2
<400> 12
   gatctagaac aaacgcactc tggagctt 28
<210> 13
   <211> 28
   <212> DNA
   <213> Artificial sequence
<220>
   <223> Description of the artificial sequence: Primer cysHex1
<400> 13
   acggtacctg agtcgcaaca atgagctt 28
<210> 14
   <211> 28
   <212> DNA
   <213> Artificial sequence
<220>
   <223> Description of the artificial sequence: Primer cysHex2
<400> 14
   gttctagacg gaggatgtgg atggattc 28
<210> 15
   <211> 28
   <212> DNA
   <213> Artificial sequence
<220>
   <223> Description of the artificial sequence: Primer cysKex1
<400> 15
   agggtaccca agcggtcgac caacaaaa 28
<210> 16
   <211> 28
   <212> DNA
   <213> Artificial sequence
<220>
   <223> Description of the artificial sequence: Primer cysKex2
<400> 16
   cttctagaat tagtcgcgga tgtcttcg 28

## Description

### Field of the Invention

The description provides nucleotide sequences from coryneform bacteria which code for the cysD, cysN, cysK, cysE and cysH genes and the invention provides a process for the fermentative preparation of amino acids using bacteria in which the endogene genes mentioned are enhanced.

### Prior Art

Z-Amino acids, in particular L-lysine, L-cysteine and L-methionine, are used in human medicine and in the pharmaceuticals industry, in the foodstuffs industry and very particularly in animal nutrition.

It is known that amino acids are prepared by fermentation from strains of coryneform bacteria, in particular Corynebacterium glutamicum. Because of their great importance, work is constantly being undertaken to improve the preparation processes. Improvements to the process can relate to fermentation measures, such as, for example, stirring and supply of oxygen, or the composition of the nutrient media, such as, for example, the sugar concentration during the fermentation, or the working up to the product form by, for example, ion exchange chromatography, or the intrinsic output properties of the microorganism itself.

Methods of mutagenesis, selection and mutant selection are used to improve the output properties of these microorganisms. Strains which are resistant to antimetabolites or are auxotrophic for metabolites of regulatory importance and produce amino acids are obtained in this manner.

Methods of the recombinant DNA technique have also been employed for some years for improving the strain of Corynebacterium strains which produce L-amino acid, by amplifying individual amino acid biosynthesis genes and investigating the effect on the amino acid production.

### Object of the Invention

The inventors had the object of providing new measures for improved fermentative preparation of L-Lysine.

### Summary of the Invention

Where L-amino acids or amino acids are mentioned in the following, this means one or more amino acids, including their salts, chosen from the group consisting of L-asparagine, L-threonine, L-serine.. L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine.

When L-lystne or lysine are mentioned in the following, not only the bases but also the salts, such as e.g. lysine monohydrochloride or lysine sulfate, are meant by this.

When L-cysteine or cysteine are mentioned in the following, the salts, such as e.g. cysteine hydrochloride or cysteine S-sulfate are also meant by this.

When L-methionine or methionine are mentioned in the following, the salts, such as e.g. methionine hydrochloride or methionine sulfate are also meant by this.

The description provides isolated polynucleotides from coryneform bacteria comprising one or more of the polynucleotide sequences which code for the cysD gene, the cysN gene, the cysK gene, the cysE gene or the cysH gene, chosen from the group consisting of
a) polynucleotide which is identical to the extent of at least 70% to a polynucleotide which codes for a polypeptide which comprises the amino acid sequence of SEQ ID No. 2,
b) polynucleotide which is identical to the extent of at least 70% to a polynucleotide which codes for a polypeptide which comprises the amino acid sequence of SEQ ID No. 3,
c) polynucleotide which is identical to the extent of at least 70% to a polynucleotide which codes for a polypeptide which comprises the amino acid sequence of SEQ ID No. 5,
d) polynucleotide which is identical to the extent of at least 70% to a polynucleotide which codes for a polypeptide which comprises the amino acid sequence of SEQ ID No. 6,
e) polynucleotide which is identical to the extent of at least 70% to a polynucleotide which codes for a polypeptide which comprises the amino acid sequence of SEQ ID No. 8,
f) polynucleotide which codes for a polypeptide which comprises an amino acid sequence which is identical to the extent of at least 70% to the amino acid sequence of SEQ ID No. 2,
g) polynucleotide which codes for a polypeptide which comprises an amino acid sequence which is identical to the extent of at least 70% to the amino acid sequence of SEQ ID No. 3,
h) polynucleotide which codes for a polypeptide which comprises an amino acid sequence which is identical to the extent of at least 70% to the amino acid sequence of SEQ ID No. 5,
i) polynucleotide, which codes for a polypeptide which comprises an amino acid sequence which is identical to the extent of at least 70% to the amino acid sequence of SEQ ID No. 6,
j) polynucleotide which codes for a polypeptide which comprises an amino acid sequence which is identical to the extent of at least 70% to the amino acid sequence of SEQ ID No. 8,
k) polynucleotide which is complementary to the polynucleotides of a), b), c), d), e), f), g), h), i) or j), and
l) polynucleotide comprising at least 15 successive nucleotides of the polynucleotide sequence of a), b), c),d), e), f), g), h), i), j) or k),
   the polypeptides preferably having the corresponding activities, namely of sulfate adenylyl transferase, cysteine synthase A, serine acetyl transferase or 3'-phopshoadenylyl sulfate reductase.

The description also provides the above-mentioned polynucleotides, these preferably being DNAs which are capable of replication, comprising:
(i) one or more nucleotide sequences shown in SEQ ID No. 1, SEQ ID No. 4 or SEQ ID No. 7, or
(ii) at least one sequence which corresponds to sequence (i) within the range of the degeneration of the genetic code, or
(iii) at least one sequence which hybridizes with the sequence complementary to sequence (i) or (ii), and optionally
(iv) sense mutations of neutral function in (i).

The description also provides
polynucleotides, in particular DNAs, which are capable of replication and comprise one or more nucleotide sequences as shown in SEQ ID No. 1, SEQ ID No.4, or SEQ ID No. 7;
polynucleotides which code for one or more polypeptides which comprises the corresponding amino acid sequences, as shown in SEQ ID No. 2, SEQ ID No.3, SEQ ID No. 5, SEQ ID No. 6, or SEQ ID No. 8;
a vector containing one or more of the polynucleotides according to the invention, in particular shuttle vectors or plasmid vectors, and
coryneform bacteria which contain the vector or in which one or more of the endogene genes chosen from the group consisting of the cysD gene, cysN gene, cysK gene, cysE gene and cysH gene is/are enhanced.

The invention provides a process for the fermentative preparation of L-Lysine using bacteria in which one or more endogene genes chosen from the group consisting of
- the cysD gene which codes for the subunit II of sulfate adenylyltransferase,
- the cyaN' gene which codes for the subunit I of sulfate adenylyl transferase,
- the cysK gene which codes for cysteine synthase A,
- the cysE gene which codes for serine acetyl transferase,
- the cysH gene which codes for 3' phosphoadenylyl sulfate reductase is overexpressed.

All five endogene genes (cysD gene, cysN gene, cysK gene, cysE gene and cysH gene) participate in the biosynthesis of the sulfur-containing L-amino acids L-cysteine and L-methionine. The carbon matrix of these amino acids is predominantly derived from the same metabolic intermediates as that of the amino acids of the aspartate family, to which h-lysirie belongs. Over-expression of one or more of the genes mentioned leads to pool shifts in the participating biosynthesis pathways, which has a positive effect on the formation of L-lysine, L-methionine and L-cysteine.

The description also provides polynucleotides which substantially comprise a polynucleotide sequence, which are obtainable by screening by means of hybridization of a corresponding gene library of a coryneform bacterium, which comprises the complete gene or parts thereof, with a probe which comprises the sequence of the polynucleotides according to the invention according to SEQ ID No. 1, SEQ ID No. 4 or SEQ ID No. 7 or a fragment thereof, and isolation of the polynucleotide sequence mentioned.

### Detailed Description of the Invention

Polynucleotides which comprise the sequences according to the invention are suitable as hybridization probes for RNA, cDNA and DNA, in order to isolate, in the full length, nucleic acids or polynucleotides or genes which code for sulfate adenylyl transferase, cysteine synthase A, serine acetyl transferase and/or 3'-phosphoadenylyl sulfate reductase, or to isolate those nucleic acids or polynucleotides or genes which have a high similarity of sequence with that of the cysD gene, the cysN gene, the cysK gene, the cysE gene and/or the cysH gene.

Polynucleotides which comprise the sequences according to the description are furthermore suitable as primers with the aid of which DNA of genes which code for sulfate adenylyl transferase, cysteine synthase A, serine acetyl transferase and/or 3'-phosphoadenylyl sulfate reductase can be prepared by the polymerase chain reaction (PCR).

In one aspect of this invention, the cysD gene according to the invention codes for the subunit II of sulfate adenylyl transferase, the cysN gene according to the invention codes for the subunit I of sulfate, adenylyl transferase, the cysK gene according to the invention codes for cysteine synthase A, the cysE gene according to the invention codes for serine acetyl transferase and the cysH gene according to the invention codes for 3'-phosphoadenylyl sulfate reductase.

In another aspect of this invention, it is possible that these genes according to the invention occur in pairs or in combination with several genes, in which case they then code for the combined activities. That is to say, if, for example, the a) cysE gene and cysK gene, or b) cysK gene and cysH gene, or c) cysN gene and cysD gene and cysE gene and cysK gene are enhanced at the same time, these code for a) serine acetyl transferase and cysteine synthase A, b) cysteine synthase A and 3'-phosphoadeny,Iyl sulfate reductase, and c) sulfate adenylyl transferase and serine acetyltransferase and cysteine synthase A.

Such oligonucleotides which serve as probes or primers comprise at least 30, preferably at least 20, very particularly preferably at least 15 successive nucleotides. Oligonucleotides which have a length of at least 40 or 50 nucleotides are also suitable. Oligonucleotides with a length of at least 100, 150, 200, 250 or 300 nucleotides are optionally also suitable.

"Isolated" means separated out of its natural environment.

"Polynucleotide"' in general relates to polyribonucleotides and polydeoxyribonucleotides, it being possible for these to be non-modified RNA or DNA or modified RNA or DNA.

The polynucleotides according to the description include a polynucleotide according to SEQ ID No. 1, SEQ ID No. 4, or SEQ ID No. 7 or a fragment prepared therefrom and also those which are at least 70%, preferably at least 80% and in particular at least 90% to 95% identical to the polynucleotide according to SEQ ID No. 1, SEQ ID No. 4 or SEQ ID No. 7 or a fragment prepared therefrom.

"Polypeptides" are understood as meaning peptides or proteins which comprise two or more amino acids bonded via peptide bonds.

The polypeptides according to the description include the polypeptides according to SEQ ID No.2, SEQ ID No. 3, SEQ ID No. 5, SEQ ID No. 6 and SEQ ID No. 8, in particular those with the biological activity of sulfate adenylyl transferase, cysteine synthase A, serine acetyl transferase and/or 3'-phosphoadenylyl sulfate reductase, and also those which are at least 70%, preferably at least 80% and in particular at least 90% to 95% identical to the polypeptides according to SEQ ID No. 2, SEQ ID No. 3, SEQ ID No. 5, SEQ ID No. 6 or SEQ ID No. 8 and have the activities mentioned.

The invention furthermore relates to a process for the fermentative preparation of L-lysine using coryneform bacteria which in particular already produce L-Lysine and in which the nucleotide sequences which code for the cysD gene, the cysN gene, cysE gene, the cysK gene and/or the cysH gene are over-expressed.

The term" enhancement" in this connection describes the increase in the intracellular activity of one or more enzymes (proteins) in a microorganism which are coded by the corresponding DNA, for example by increasing the number of copies of the gene or genes, using a potent promoter or using a gene or allele which codes for a corresponding enzyme (protein) having a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

The microorganisms which the present description provides can produce L-amino acids from glucose, sucrose, lactose, fructose, maltose, molasses, starch, cellulose or from glycerol and ethanol. They can be representatives of coryneform bacteria, in particular of the genus Corynebacterium. Of the genus Corynebacterium, there may be mentioned in particular the species Corynebacterium glutamicum, which is known among experts for its ability to produce L-amino acids.

Suitable strains of the genus Corynebacterium, in particular of the species Corynebacterium glutamicum (C. glutamicum), are in particular the known wild-type strains
Corynebacterium glutamicum ATCC13032
Corynebacterium acetoglutamicum ATCC1S806
Corynebacterium acetoacidophilum ATCC13870
Corynebacterium thermoaminogenes FERM BP-1539
Corynebacterium melassecola ATCC27965
Brevibacterium flavum ATCC14067
Brevibacterium lactofermentum ATCC13869 and
Brevibacterium divaricatum ATCC14020
and L-Lysine producing mutants or strains prepared therefrom.

The new cysD, cysN, cysK, cysE and cysH genes of C. glutamicum which code for the enzymes sulfate adenylyl transferase (EC 2.7.7.4), cysteine synthase A (EC 4.2.99.8), serine acetyl transferase (EC 2.3.1.30) and 3'-phosphoadenylyl sulfate reductase (EC 1.8.99.4) have been isolated.

To isolate the cysD gene, the cysN gene, the cysK gene, the cysE gene, the cysH gene or also other genes of C. glutamicum, a gene library of this microorganism is first set up in Escherichia coli (E. coli). The setting up of gene libraries is described in generally known-textbooks and handbooks. The textbook by Winnacker: Gene und Klone, Eine Einführung in die Gentechnologie (Verlag Chemie, Weinheim, Germany, 1990), or the handbook by sambrook et al.: Molecular Cloning, A Laboratory Manual (Cold Spring Harbor Laboratory Press, 1989) may be mentioned as an example. A well-known gene library is that of the E. coli K-12 strain W3110 set up in λ vectors by Kohara et al. (Cell 50, 495 -508 (1987)). Bathe et al. (Molecular and General Genetics, 252:255-265, 1996) describe a gene library of C. glutamicum ATCC13032, which was set up with the aid of the cosmid vector SuperCos I (Wahl et al., 1987, Proceedings of the National Academy of Sciences USA, 84:2160-2164) in the E. coli K-12 strain NM554 (Raleigh et al., 1988, Nucleic Acids Research 16:1563-1575).

Börmann et al. (Molecular Microbiology 6(3), 317-326) (1992)) in turn describe a gene library of C. glutamicum ATCC13032 using the cosmid pHC79 (Hohn and Collins, Gene 11, 291-298 (1980)).

To prepare a gene library of C. glutamicum in E. coli it is also possible to use plasmids such as pBR322 (Bolivar, Life Sciences, 25, 807-818 (1979)) or pUC9 (Vieira et al., 1982, Gene, 19:259-268). Suitable hosts are, in particular, those E. coli strains which are restriction- and recombination-defective. An example of these is the strain DH5αmcr, which has been described by Grant et al. (Proceedings of the National Academy of Sciences USA, 87 (1990) 4645-4649). The long DNA fragments cloned with the aid of cosmids can in turn be subcloned in the usual vectors suitable for sequencing and then sequenced, as is described e.g. by Sanger et al. (Proceedings of the National Academy of Sciences of the United States of America, 74:5463-5467, 1977).

The resulting DNA sequences can then be investigated with known algorithms or sequence analysis programs, such as e. g. that of Staden (Nucleic Acids Research 14, 217-232(1986)), that of Marck (Nucleic Acids Research 16, 1829-1836 (1988)) or the GCG program of Butler (Methods of Biochemical Analysis 39, 74-97 (1998)).

The DNA sequences of C. glutamicum which code for the cysD, cysN, cysK, cysE and cysH genes and which, as SEQ ID No. 1, SEQ ID No. 4, and SEQ ID No. 7, are constituents of the present description have been found. The amino acid sequence of the corresponding proteins has furthermore been derived from the present DNA sequences by the methods described above. The resulting amino acid sequences of the cysD, cysN, cysK, cysE and cysH gene products are shown in SEQ ID NO. 2, SEQ ID No. 3, SEQ ID No. 5, SEQ ID No. 6 and SEQ ID No. 8.

Coding DNA, sequences which result from SEQ ID No.1, SEQ ID No. 4 or SEQ ID No. 7 by the degeneracy of the genetic code are also a constituent of the description. In the same way, DNA sequences which hybridize with SEQ ID No.1 or parts of SEQ ID No. 1 or SEQ ID No. 4 or parts of SEQ ID No. 4 or SEQ ID No.7 or parts of SEQ ID No.7 are a constituent of the description. Conservative amino acid exchanges, such as e.g. exchange of glycine for alanine or of aspartic acid for glutamic acid in proteins, are furthermore known among experts as "sense mutations" which do not lead to a fundamental change in the activity of the protein, i. e. are of neutral function. It is furthermore known that changes on the N and/or C terminus of a protein cannot substantially impair or can even stabilize the function thereof. Information in this context can be found by the expert, inter alia, in Ben-Bassat et al. (Journal of Bacteriology 169:751-757 (1987)), in O'Regan et al. (Gene 77:237-251 (1989)), in Sahin-Toth at al. (Protein Sciences 3:240-247 (1994)), in Hochuli et al. (Bio/Technology 6:1321-1325 (1988)) and in known textbooks of genetics and molecular biology. Amino acid sequences which result in a corresponding manner from SEQ ID No. 2, SEQ ID No. 3, SEQ ID No. 5, SEQ ID No. 6 and SEQ ID No. 8 are also a constituent of the description.

In the same way, DNA sequences which hybridize with SEQ ID No. 1 or parts of SEQ ID No.1 or SEQ ID No.4 or parts of SEQ ID No.4 or SEQ ID No.7 or parts of SEQ ID No. 7 are a constituent of the ID No.7 or parts of SEQ ID DNA sequences which are prepared by the polymerase chain reaction (PCR) using primers which result from SEQ ID No.1, SEQ ID No. 4 or SEQ ID No - 7 are a constituent of the description. Such oligonucleotides typically have a length of at least 15 nucleotides.

Instructions for identifying DNA sequences by means of hybridization can be found by the expert, inter alia, in the handbook "The DIG System Users Guide for Filter Hybridization" from Boehringer Mannheim GmbH (Mannheim, Germany, 1993) and in Liebl et al. (International Journal of Systematic Bacteriology (1991) 41: 255-260). The hybridization takes place under stringent conditions, that is to say only hybrids in which the probe and target sequence, i. e. the polynucleotides treated with the probe, are at least 70% identical are formed. It is known that the stringency of the hybridization, including the washing steps, is influenced or determined by varying the buffer composition, the temperature and the salt concentration. The hybridization reaction is preferably carried out under a relatively low stringency compared with the washing steps (Hybaid Hybridisation Guide, Hybaid Limited, Teddington, UK, 1996).

A 5x SSC buffer at a temperature of approx. 50°C - 68°C, for example, can be employed for the hybridization reaction. Probes can also hybridize here with polynucleotides which are less than 70% identical to the sequence of the probe. Such hybrids are less stable and are removed by washing under stringent conditions. This can be achieved, for example, by lowering the salt concentration to 2x SSC and optionally subsequently 0.5x SSC (The DIG System User's Guide for Filter Hybridisation, Boehringer Mannheim, Mannheim, Germany, 1995) a temperature of approx. 50°C - 68°C being established. It is optionally possible to lower the salt concentration to 0.1x SSC. Polynucleotide fragments which are, for example, at least 70% or at least 80% or at least 90% to 95% identical to the sequence of the probe employed can be isolated by increasing the hybridization temperature stepwise from 50°C to 68°C in steps of approx. 1 - 2°C. Further instructions on hybridization are obtainable on the market in the form of so-called kits (e.g. DIG Easy Hyb from Roche Diagnostics GmbH, Mannheim, Germany, Catalogue No. 1603558).

Instructions for amplification of DNA sequences with the aid of the polymerase chain reaction (PCR) can be found by the expert, inter alia, in the handbook by Gait: Oligonucleotide Synthesis: A Practical Approach (IRL Press, Oxford, UK, 1984) and in Newton and Graham: PCR (Spektrum Akademischer Verlag, Heidelberg, Germany, 1994).

It has been found that coryneform bacteria produce L-lysine in an improved manner after over-expression of one or more of the genes chosen from the group consisting of the cysD gene, cysN gene, cysK gene, cysE gene and cysH gene.

To achieve an over-expression, the number of copies of the corresponding genes can be increased, or the promoter and regulation region or the ribosome binding site upstream of the structural gene can be mutated. Expression cassettes which are .incorporated upstream of the structural gene act in the same way. By inducible promoters, it is additionally possible to ,increase the expression in the course of fermentative amino acid production. The expression is likewise improved by measures to prolong the life of the m-RNA. Furthermore, the enzyme activity is also increased by preventing the degradation of the enzyme protein. The genes or gene constructs can either be present in plasmids with a varying number of copies, or can be integrated and amplified in the chromosome, alternatively, an over-expression of the genes in question can furthermore be achieved by changing the composition of the media and the culture procedure.

Instructions in this context can be found by the expert, inter alia, in Martin et al. (Bio/Technology 5, 7.37-146 (1987)), in Guerrero et al. (Gene 138, 35-41 (1994)), Tsuchiya and Morinaga (Bio/Technology 6, 428-430 (1988)), in Eikmanns et al. (Gene 102, 93-98 (1991)), in EP 0 472 869, in US 4,601,893, in Schwarzer and Pühler (Bio/Technology 9, 84-87 (1991), in Reinscheid et al. (Applied and Environmental Microbiology 60, 126-132 (1994)), in LaBarre et al. (Journal of Bacteriology 175, 1001-1007 (1993)), in WO 96/15246, in Malumbres et al. (Gene 134, 15 - 24 (1993)), in JP-A-10-229891, in Jensen and Hammer (Biotechnology and Bioengineering 58, 191-195 (1998)), in Makrides (Microbiological Reviews 60:512-538 (1996)) and in known textbooks of genetics and molecular biology.

By way of example, for enhancement the cysD, cysN, cysK, cysE or cysH genes according to the invention were over-expressed with the aid of episomal plasmids. Suitable plasmids are those which are replicated in coryneform bacteria. Numerous known plasmid vectors, such as e.g. pZ1 (Menkel et al., Applied and Environmental Microbiology (1989) 64: 549-554), pEKExl (Eikmanns et al., Gene 102:93-98 (1991)) or pHS2-1 (Sonnen et al., Gene 107:69-74 (1991)) are based on the cryptic plasmids pHM1519, pBL1 or pGA1. Other plasmid vectors, such as e.g. those based on pCG4 (US-A 4,489,160), or pNG2 (Serwold-Davis et al., FEMS Microbiology Letters 66, 119-124 (1990)), or pAG1 (US-A 5,158,891), can be used in the same manner.

Plasmid vectors which are furthermore suitable are also those with the aid of which the process of gene amplification by integration into the chromosome can be used, as has been described, for example, by Reinscheid et al. (Applied and Environmental Microbiology 60, 126-132 (1994)) for duplication or amplification of the hom-thrB operon. In this method, the complete gene is cloned in a plasmid vector which can replicate in a host (typically E. coli), but not in C. glutamicum. Possible vectors are, for example, pSUP301 (Simon et al., Bio/Technology 1, 784-791 (1983)), pK18mob or pK19mob (Schäfer et al., Gene 145, 69-73 (1994)), pGEM-T (Promega Corporation, Madison, WI, USA), pCR2.1-TOPO (Shuman (1994). Journal of Biological Chemistry 269:32678-84; US-A 5,487,993), pCR®Blunt (Invitrogen, Groningen, Holland; Bernard et al., Journal of Molecular Biology, 234: 534-541 (1993)), pEM1 (Schrumpf et al, 1991, Journal of Bacteriology 173:4510-4516) or pBGS8 (Spratt et al., 1986, Gene 41: 337-342). The plasmid vector which contains the gene to be amplified is then transferred into the desired strain of C. glutamicum by conjugation or transformation. The method of conjugation is described, for example, by Schäfer et al. (Applied and Environmental Microbiology 60, 756-759 (1994)). Methods for transformation are described, for example, by Thierbach et al. (Applied Microbiology and Biotechnology 29, 356-362 (1988)), Dunican and Shivnan (Bio/Technolo.gy 7, 1067-1070 (1989)) and Tauch et al. (FEMS Microbiological Letters 123, 343-347 (1994)). After homologous recombination by means of a "cross over" event, the resulting strain contains at least two copies of the gene in question.

In addition, it may be advantageous for the production of L-Lysine to over-express one or more enzymes of the particular biosynthesis pathway, of glycolysis, of anaplerosis, of the citric acid cycle, of the pentose phosphate cycle, of amino acid export and optionally regulatory proteins, in addition to the cysD gene, the cysN gene, the cysK gene, the cysE gene and/or the cysH gene.

Thus, for the preparation of L-amino acids, in addition to enhancement of the cysD gene, the cysN gene, the cysK gene, the cysE gene and/or the cysH gene, one or more endogene genes chosen from the group consisting of
- the dapA gene which codes for dihydrodipicolinate synthase (EP--B 0 197 335),
- the gap gene which codes for glyceraldehyde 3-phosphate dehydrogenase (Eikmanns (1992), Journal of Bacteriology 174:6076-6086),
- the tpi gene which codes for triose phosphate isomerase (Eikmanns (1992), Journal of Bacteriology 174:6076-6086),
- the pgk gene which codes for 3-phosphoglycerate kinase (Eikmanns (1992), Journal of Bacteriology 174:6076-6086),
- the zwf gene which codes for glucose 6-phosphate dehydrogenase (JP-A-09224661),
- the pyc gene which codes for pyruvate carboxylase (DE-A-198 31 609),
- the mqo gene which codes for malate-quinone oxidoreductase (Molenaar et al., European Journal of Biochemistry 254, 395-403 (1998)),
- the lysC gene which codes for a feed-back resistant aspartate kinase (Accession No.P26512; EP-B-0387527; EP-A-0699759),
- the lysE gene which codes for lysine export (DE-A-195 48 222),
- the hom gene which codes for homoserine dehydrogenase (EP-A 0131171),
- the ilvA gene which codes for threonine dehydratase (Möckel et al., Journal of Bacteriology (1992) 8065-8072)) or the ilvA(Fbr) allele which codes for a "feed back resistant" threonine dehydratase (Möckel et al., (1994) Molecular Microbiology 13: 833-842),
- the ilvBN gene which codes for acetohydroxy-acid synthase (EP-B 0356739),
- the ilvD gene which codes for dihydroxy-acid dehydratase (Sahm and Eggeling (1999) Applied and Environmental Microbiology 65: 1973-1979),
- the zwa1 gene which codes for the Zwal protein (DE: 19959328.0, DSM 13115)
can be in particular over-expressed.

It may furthermore be advantageous for the production of L-Lysine, in addition to enhancement of the cysD gene, the cysN gene, the cysK gene, the cysE gene and/or the cysH gene, for one or more genes chosen from the group consisting of
- the pck gene which codes for phosphoenol pyruvate carboxykinase (DE 199 50 409.1; DSM 13047),
- the pgi gene which codes for glucose 6-phosphate isomerase (US 09/396,478; DSM 12969),
- the poxB gene which codes for pyruvate oxidase (DE: 1995 1975.7; DSM 13114),
- the zwa2 gene which codes for the Zwa2 protein (DE : 19959327.2, DSM 13113)
to be attenuated, in particular for the expression thereof to be reduced. For the production of L-cysteine in particular, it may be advantageous, in addition to enhancement of the cysD gene, the cysN gene, the cysK gene, the cysE gene and/or the cysH gene, for one or more genes chosen from the group consisting of
- the aecD gene which codes for cystathionine β-lyase (Accession Number M89931 des National Center for Biotechnology Information (NCBI, Bethesda, MD, USA),
- the metB gene which codes for cystathione γ-synthase (Accession Number AF1236953 des National Center for Biotechnology Information (NCBI, Bethesda, MD, USA)
to be attenuated, in particular for the expression thereof to be reduced.

The term "attenuation" in this connection describes the reduction or elimination of the intracellular activity of one or more enzymes (proteins) in a microorganism which are coded by the corresponding DNA, for example by using a weak promoter or using a gene or allele which codes for a corresponding enzyme with a low activity or inactivates the corresponding gene or enzyme (protein), and optionally combining these measures.

By attenuation measures, the activity or concentration of the corresponding protein is in general reduced to 0 to 75%, to 50%, 0 to 25%, 0 to 10% or 0 to 5% of the activity or concentration of the wild-type protein or of the activity or concentration of the protein in the starting microorganism.

In addition to over-expression of the cysD gene, the cysN gene, the cysK gene, the cysE gene and/or the cysH gene it may furthermore be advantageous for the production of amino acids to eliminate undesirable side reactions (Nakayama: "Breeding of Amino Acid Producing Micro-organisms", in: Overproduction of Microbial Products, Krumphanzl, Sikyta, Vanek (eds.), Academic Press, London, UK, 1982).

The description also provides the microorganisms prepared according to the invention, and these can be cultured continuously or discontinuously in the batch process (batch culture) or in the fed batch (feed process) or repeated fed batch process (repetitive feed process) for the purpose of production of amino acids. A summary of known culture methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren und periphere Einrichtungen (Vieweg Verlag, Braunschweig/Wiesbaden, 1994)).

The culture medium to be used must meet the requirements of the particular strains in a suitable manner. Descriptions of culture media for various microorganisms are contained in the handbook "Manual of Methods for General Bacteriology" of the American Society for Bacteriology (Washington D.C., USA, 1981).

Sugars and carbohydrates, such as e.g. glucose, sucrose, lactose, fructose, maltose, molasses, starch and cellulose, oils and fats, such as e.g. soya oil, sunflower oil, groundnut oil and coconut fat, fatty acids, such as e.g. palmitic acid, stearic acid and linoleic acid, alcohols, such as e.g. glycerol and ethanol, and organic acids, such as e.g. acetic acid, can be used as the source of carbon. These substances can be used individually or as a mixture.

Organic nitrogen-containing compounds, such as peptones, yeast extract, meat extract, malt extract, corn steep liquor, soya bean flour and urea, or inorganic compounds, such as ammonium sulfate, ammonium chloride, ammonium phosphate, ammonium carbonate and ammonium nitrate, can be used as the source of nitrogen. The sources of nitrogen can be used individually or as a mixture.

Phosphoric acid, potassium dihydrogen phosphate or dipotassium hydrogen phosphate or the corresponding sodium-containing salts can be used as the source of phosphorus.

Organic and inorganic sulfur-containing compounds, such as, for example, sulfides, sulfites, sulfates and thiosulfates, can be used as a source of sulfur, in particular for the preparation of sulfur-containing amino acids.

The culture medium must furthermore comprise salts of metals, such as e. g. magnesium sulfate or iron sulfate, which are necessary for growth. Finally, essential growth substances, such as amino acids and vitamins, can be employed in addition to the above-mentioned substances.

Suitable precursors can moreover be added to the culture medium. The starting substances mentioned can be added to the culture in the form of a single batch, or can be fed in during the culture in a suitable manner.

Basic compounds, such as sodium hydroxide, potassium hydroxide, ammonia or aqueous ammonia, or acid compounds, such as phosphoric acid or sulfuric acid, can be employed in a suitable manner to control the pH of the culture. Antifoams, such as e.g. fatty acid polyglycol esters, can be employed to control the development of foam. Suitable substances having a selective action, such as e.g. antibiotics, can be added to the medium to maintain the stability of plasmids. To maintain aerobic conditions, oxygen or oxygen-containing gas mixtures, such as e.g. air, are introduced into the culture. The temperature of the culture is usually 20°C to 45°C, and preferably 25°C to 40°C. Culturing is continued until a maximum of the desired product has formed. This target is usually reached within 10 hours to 160 hours.

The fermentation broths obtained in this way, in particular containing L-methionine, usually have a dry weight of 7.5 to 25 wt.% and contain L-methionine. It is furthermore also advantageous if the fermentation is conducted in a sugar-limited procedure at least at the end, but in particular over at least 30% of the duration of the fermentation. That is to say, the concentration of utilizable sugar in the fermentation medium is reduced to ≥ 0 to 3 g/l during this period.

The fermentation broth prepared in this manner, in particular containing L-methionine, is then further processed. Depending on requirements, the all or some of the biomass can be removed from the fermentation broth by separation methods, such as e.g. centrifugation, filtration, decanting or a combination thereof, or it can be left completely in this. This broth is then thickened or concentrated by known methods, such as e.g. with the aid of a rotary evaporator, thin film evaporator, falling film evaporator, by reverse osmosis, or by nanofiltration. This concentrated fermentation broth can then be worked up by methods of freeze drying, spray drying, spray granulation or by other processes to give a preferably free-flowing, finely divided powder.

This free-flowing, finely divided powder can then in turn by converted by suitable compacting or granulating processes into a coarse-grained, readily free-flowing, storable and largely dust-free product. In the granulation or compacting it is advantageous to employ conventional organic or inorganic auxiliary substances or carriers, such as starch, gelatin, cellulose derivatives or similar substances, such as are conventionally used as binders, gelling agents or thickeners in foodstuffs or feedstuffs processing, or further substances, such as, for example, silicas, silicates or stearates.

"Free-flowing" is understood as meaning powders which flow unimpeded out of the vessel with the opening of 5 mm (millimeters) of a series of glass outflow vessels with outflow openings of various sizes (Klein, Seifen, Öle, Fette, Wachse 94, 12 (1968)).

As described here, "finely divided" means a powder with a predominant content (> 50 %) with a particle size of 20 to 200 µm diameter. "Coarse-grained" means products with a predominant content (> 50 %) with a particle size of 200 to 2000 µm diameter. In this context, "dust-free" means that the product contains only small contents (< 5 %) with particle sizes of less than 20 µm diameter. The particle size determination can be carried out with methods of laser diffraction spectrometry. The corresponding methods are described in the textbook on "Teilchengrößenmessung in der Laborpraxis" by R. H. Müller and R. Schuhmann, Wissenschaftliche Verlagsgesellschaft Stuttgart (1996) or in the textbook "Introduction to Particle Technology" by M. Rhodes, Verlag Wiley & Sons (1998).

"Storable" in the context of this invention means a product which can be stored for up to 120 days, preferably up to 52 weeks, particularly preferably 60 months, without a substantial loss (< 5%) of methionine occurring.

Alternatively, however, the product can be absorbed on to an organic or inorganic carrier substance which is known and conventional in feedstuffs processing, such as, for example, silicas, silicates, grits, brans, meals, starches, sugars or others, and/or mixed and stabilized with conventional thickeners or binders. Use examples and processes in this context are described in the literature (Die Mühle + Mischfuttertechnik 132 (1995) 49, page 817).

Finally, the product can be brought into a state in which it is stable to digestion by animal stomachs, in particular the stomach of ruminants, by coating processes ("coating") using film-forming agents, such as, for example, metal carbonates, silicas, silicates, alginates, stearates, starches, gums and cellulose ethers, as described in DE-C-4100920.

If the biomass is separated off during the process, further inorganic solids, for example added during the fermentation, are in general removed. In addition, the animal feedstuffs additive according to the invention comprises at least the predominant proportion of the further substances, in particular organic substances, which are formed or added and are present in solution in the fermentation broth, where these have not been separated off by suitable processes.

In one aspect of the invention, the biomass can be separated off to the extent of up to 70%, preferably up to 80%, preferably up to 90%, preferably up to 95%, and particularly preferably up to 100%. In another aspect of the invention, up to 20% of the biomass, preferably up to 15%, preferably up to 10%, preferably up to 5%, particularly preferably no biomass is separated off.

These organic substances include organic by-products which are optionally produced, in addition to the L-methionine, and optionally discharged by the microorganisms employed in the fermentation. These include L-amino acids chosen from the group consisting of L-lysine, L-valine, L-threonine, L-alanine or L-tryptophan. They include vitamins chosen from the group consisting of vitamin B1 (thiamine), vitamin B2 (riboflavin),vitamin B5 (pantothenic acid), vitamin B6 (pyridoxine), vitamin B12 (cyanocobalamin), nicotinic acid/nicotinamide and vitamin E (tocopherol). They include furthermore organic acids which carry one to three carboxyl groups, such as, for example, acetic acid, lactic acid, citric acid, malic acid or fumaric acid. Finally, they also include sugars, such as, for example, trehalose. These compounds are optionally desired if they improve the nutritional value of the product.

These organic substances, including L-methionine and/or D-methionine and/or the racemic mixture D,L-methionine, can also be added, depending on requirements, as a concentrate or pure substance in solid or liquid form during a suitable process step. These organic substances mentioned can be added individually or as mixtures to the resulting or concentrated fermentation broth, or also during the drying or granulation process. It is likewise possible to add an organic substance or a mixture of several organic substances to the fermentation broth and a further organic substance or a further mixture of several organic substances during a later process step, for example granulation.

The product described above is suitable as a feedstuffs additive, i.e. feed additive, for animal nutrition.

The L-methionine content of the animal feedstuffs additive is conventionally 1 wt.% to 80 wt.%, preferably 2 wt.% to 80 wt.%, particularly preferably 4 wt.% to 80 wt.%, and very particularly preferably 8 wt.% to 80 wt.%, based on the dry weight of the animal feedstuffs additive. Contents of 1 wt.% to 60 wt.%, 2 wt.% to 60 wt.%, 4 wt.% to 60 wt.%, 6 wt.% to 60 wt.%, 1 wt.% to 40 wt.%, 2 wt.% to 40 wt.% or 4 wt.% to 40 wt.% are likewise possible. The water content of the feedstuffs additive is conventionally up to 5 wt.%, preferably up to 4 wt.%, and particularly preferably less than 2 wt.%.

The description accordingly also provides a process for the preparation of an L-methionine-containing animal feedstuffs additive from fermentation broths, which comprises the steps
a) culture and fermentation of an L-methionine-producing microorganism in a fermentation medium;
b) removal of water from the L-methionine-containing fermentation broth (concentration) ;
c) removal of an amount of 0 to 100 wt.% of the biomass formed during the fermentation; and
d) drying of the fermentation broth obtained according to a) and/or b) to obtain the animal foodstuffs additive in the desired powder or granule form.
   If desired, one or more of the following steps can furthermore be carried out in the process according to the invention:
e) addition of one or more organic substances, including li-methionine and/or D-methionine and/or the racemic mixture D,L-methionine, to the products obtained according to a), b) and/or c);
f) addition of auxiliary substances chosen from the group consisting of silicas, silicates, stearates, grits and bran to the substances obtained according to a) to d) for stabilization and to increase the storability; or
g) conversion of the substances obtained according to a) to e) into a form which is stable in an animal stomach, in particular rumen, by coating with film-forming agents.

Methods for the determination of L-amino acids are known from the prior art. The analysis can thus be carried out, for example, as described by Spackman et al. (Analytical Chemistry, 30, (1958), 1190) by ion exchange chromatography with subsequent ninhydrin derivation, or it can be carried out by reversed phase HPLC, for example as described by Lindroth et al. (Analytical Chemistry (1979) 51: 1167-1174).

The process according to the invention is used for fermentative preparation of L-Lysine.

The following microorganisms were deposited as a pure culture on 18th May 2001 at the Deutsche Sammlung für Mikroorganismen und Zellkulturen (DSMZ = German collection of Microorganisms and Cell Cultures, Braunschweig, Germany) in accordance with the Budapest Treaty:
- E. coli DHSamcr/pEC-XK99EcysEb2ex as DSM 14308,
- E. coli DH5amcr/pEC-XK99EcysKalex as DSM 14310,
- E. coli DH5oancr/pEC-XK99EcysDa1ex as DSM 1433.1,
- E. coli DH5αmcr/pEC-XK99EcysHalex as DSM 14315.

The present invention is explained in more detail in the following with the aid of embodiment examples.

The isolation of plasmid DNA from Escherichia coli and all techniques of restriction, Klenow and alkaline phosphatase treatment were carried out by the method of Sambrook et al. (Molecular Cloning. A Laboratory Manual (1989) Cold Spring Harbour Laboratory Press, Cold Spring Harbor, NY, USA). Methods for transformation of Escherichia coli are also described in this handbook.

The composition of the usual nutrient media, such as LB or TY medium, can also be found in the handbook by Sambrook et al.

### Example 1

### Preparation of a genomic cosmid gene library from Corynebacterium glutamicum ATCC 13032

Chromosomal DNA from Corynebacterium glutamicum ATCC 13032 was isolated as described by Tauch et al. (1995, Plasmid 33:168-179) and partly cleaved with the restriction enzyme Sau3AI (Amersham Pharmacia, Freiburg, Germany, Product Description Sau3AI, Code no. 27-0913-02). The DNA fragments were dephosphorylated with shrimp alkaline phosphatase (Roche Diagnostics GmbH, Mannheim, Germany, Product Description SAP, Code no. 1758250). The DNA of the cosmid vector SuperCosl (Wahl et al. (1987) Proceedings of the National Academy of Sciences USA 84:2160-2164), obtained from Stratagene (La Jolla, USA, Product Description SuperCos1 Cosmid Vector Kit, Code no. 251301) was cleaved with the restriction enzyme XbaI (Amersham Pharmacia, Freiburg, Germany, Product Description XbaI, Code no. 27-0948-02) and likewise dephosphorylated with shrimp alkaline phosphatase.

The cosmid DNA was then cleaved with the restriction enzyme BamHI (Amersham Pharmacia, Freiburg, Germany, Product Description BamHI, Code no. 27-0868-04). The cosmid DNA treated in this manner was mixed with the treated ATCC13032 DNA and the batch was treated with T4 DNA ligase (Amersham Pharmacia, Freiburg, Germany, Product Description T4-DNA-Ligase, Code no.27-0870-04). The ligation mixture was then packed in phages with the aid of Gigapack II XL Packing Extract (Stratagene, La Jolla, USA, Product Description Gigapack II XL Packing Extract, Code no. 200217).

For infection of the E. coli strain NM554 (Raleigh et al. 1988, Nucleic Acid Research 16:1563-1575) the cells were taken up in 10 mM MgSO₄ and mixed with an aliquot of the phage suspension. The infection and titering of the cosmid library were carried out as described by Sambrook et al. (1989, Molecular Cloning: A laboratory Manual, Cold Spring Harbor), the cells being plated out on LB agar (Lennox, 1955, Virology, 1:190) with 100 mg/l ampicillin. After incubation overnight at 37°C, recombinant individual clones were selected.

### Example 2

### Isolation and sequencing of the cysD gene, the cysN gene, the cysK gene, the cysE gene or the cysH gene

The cosmid DNA of an individual colony was isolated with the Qiaprep Spin Miniprep Kit (Product No. 27106, Qiagen, Hilden, Germany) in accordance with the manufacturer's instructions and partly cleaved with the restriction enzyme Sau3AI (Amersham Pharmacia, Freiburg, Germany, Product Description Sau3AI, Product No. 27-0913-02). The DNA fragments were dephosphorylated with shrimp alkaline phosphatase (Roche Diagnostics GmbH, Mannheim, Germany, Product Description SAP, Product No. 1758250). After separation by gel electrophoresis, the cosmid fragments in the size range of 1500 to 2000 bp were isolated with the QiaExII Gel Extraction Kit (Product No. 20021, Qiagen, Hilden, Germany).

The DNA of the sequencing vector pZero-1, obtained from Invitrogen (Groningen, Holland, Product Description Zero Background Cloning Kit, Product No. K2500-01), was cleaved with the restriction enzyme BamHI (Amersham Pharmacia, Freiburg, Germany, Product Description BamHI, Product No. 27-0868-04). The ligation of the cosmid fragments in the sequencing vector pZero-1 was carried out as described by Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then electroporated (Tauch et al. 1994, FEMS Microbiol Letters, 123:343-7) into the E. coli strain DH5aMCR (Grant, 1990, Proceedings of the National Academy of Sciences U.S.A., 87:4645-4649) and plated out on LB agar (Lennox, 1955, Virology, 1:190) with 50 mg/l zeocin.

The plasmid preparation of the recombinant clones was carried out with the Biorobot 9600 (Product No. 900200, Qiagen, Hilden, Germany). The sequencing was carried out by the dideoxy chain termination method of Sanger et al. (1977, Proceedings of the National Academy of .Sciences U.S.A., 74:5463-5467) with modifications according to Zimmermann et al. (1990, Nucleic Acids Research, 18:1067). The"RR dRhodamin Terminator Cycle Sequencing Kit" from PE Applied Biosystems (Product No. 403044, Weiterstadt, Germany) was used. The separation by gel electrophoresis and analysis of the sequencing reaction were carried out in a "Rotiphoresis NF Acrylamide/Bisacrylamide" Gel (29:1) (Product No. A124.1, Roth, Karlsruhe, Germany) with the "ABI Prism 377" sequencer from PE Applied Biosystems (Weiterstadt, Germany).

The raw sequence data obtained were then processed using the Staden program package (1986, Nucleic Acids Research, 14:217-231) version 97-0. The individual sequences of the pZero1 derivatives were assembled to a continuous contig.

The computer-assisted coding region analysis was prepared with the XNIP program (Staden, 1986, Nucleic Acids Research, 14:217-231).

The resulting nucleotide sequences are shown in SEQ ID No. 1 SEQ ID No. 4 and SEQ ID No. 7. Analysis of the nucleotide sequences showed six open reading frames of 915 base pairs, 1302 base pairs, 936 base pairs, 567 base pairs and 786 base pairs, which were called the cysD gene, cysN gene, cysK gene, cysE gene and cysH gene. The cysD gene codes for a protein of 304 amino acids, the cysN gene codes for a protein of 433 amino acids, the cysK gene codes for a protein of 311 amino acids, the cysE gene codes for a protein of 188 amino acids and the cysH gene codes for a protein of 261 amino acids.

### Example 3

### Preparation of shuttle expression vectors based on pEC-XK99E for enhancement of the cysD, cysK, cysE and cysH genes in C. glutamicum

### 3.1 Amplification of the cysD, cysK, cysE and cysH genes

From the strain ATCC 13032, chromosomal DNA was isolated by the method of Eikmanns et al. (Microbiology 140: 1817-1828 (1994)). On the basis of the sequences of the cysD, cysK, cysE and cysH genes known for C. glutamicum from Example 2, the following oligonucleotides, listed in Table 1, were chosen for the polymerase chain reaction (see SEQ ID No. 9, SEQ ID No. 10, SEQ ID No. 11, SEQ ID No. 12, SEQ ID No. 13, SEQ ID No. 14, SEQ ID No. 15 and SEQ ID No. 16). In addition, suitable restriction cleavage sites which allow cloning into the target vector were inserted into the primers. They are listed in Table 1 and identified by underlining in the nucleotide sequence.

**Table 1**

| Primer | Sequence with restriction cleavage site | Amplified fragment |
|---|---|---|
| cysDex1 | | cysD (1017 bp) |
| cysDex2 | | |
| cysKex1 | | cysK (1005 bp) |
| cysKex2 | | |
| cysEex1 | | cysE (672 bp) |
| cysEex2 | | |
| cysHex1 | | cysH (884 bp) |
| cysHex2 | | |

The primers shown were synthesized by MWG-Biotech AG (Ebersberg, Germany) and the PCR reaction was carried out by the standard PCR method of Innis et al. (PCR Protocols. A Guide to Methods and Applications, 1990, Academic Press) with Pwo-Polymerase from Roche Diagnostics GmbH (Mannheim, Germany). With the aid of the polymerase chain reaction, the primers allow amplification of a DNA fragment 1017 bp in size, which carries the cysD gene, and a DNA fragment 1005 bp in size, which carries the cysK gene, a DNA fregment 672 bp in size, which carries the cysE gene, and a DNA fragment 884 bp in size, which carries the cysH gene. The cysD fragment, the cysK fragment, the cysE fragment and the cysH fragment were cleaved with the restriction endonucleases KpnI and XbaI and then isolated from the agarose gel with the QiaExII Gel Extraction Kit (Product No. 20021, Qiagen, Hilden, Germany).

### 3.2 Construction of the shuttle vector pEC-XK99E

The E. coli - C. glutamicum shuttle vector pEC-XK99E was constructed according to the prior art. The vector contains the replication region rep of the plasmid pGA1 including the replication effector per (US-A- 5,175,108; Nesvera et al., Journal of Bacteriology 179, 1525-1532 (1997)), the kanamycin resistance gene aph(3')-IIa from Escherichia coli (Beck et al. (1982), Gene 19: 327-336), the replication origin of the trc promoter, the termination regions T1 and T2, the lacI^{q} gene (repressor of the lac operon of E. coli) and a multiple cloning site (mcs) (Norrander, J.M. et al. Gene 26, 101-106 (1983)) of the plasmid pTRC99A (Amann et al. (1988), Gene 69: 301-315).

The E. coli - C. glutamicum shuttle vector pEC-XK99E constructed was transferred into C. glutamicum DSM5715 by means of electroporation (Liebl et al., 1989, FEMS Microbiology Letters, 53:299-303). Selection of the transformants took place on LBHIS agar comprising 18.5 g/l brain-heart infusion broth, 0.5 M sorbitol, 5 g/l Bactotryptone, 2.5 g/l Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from a transformant by conventional methods (Peters-Wendisch et al., 1998, Microbiology, 144, 915 - 927), cleaved with the restriction endonuclease HindIII, and the plasmid was checked by subsequent agarose gel electrophoresis.

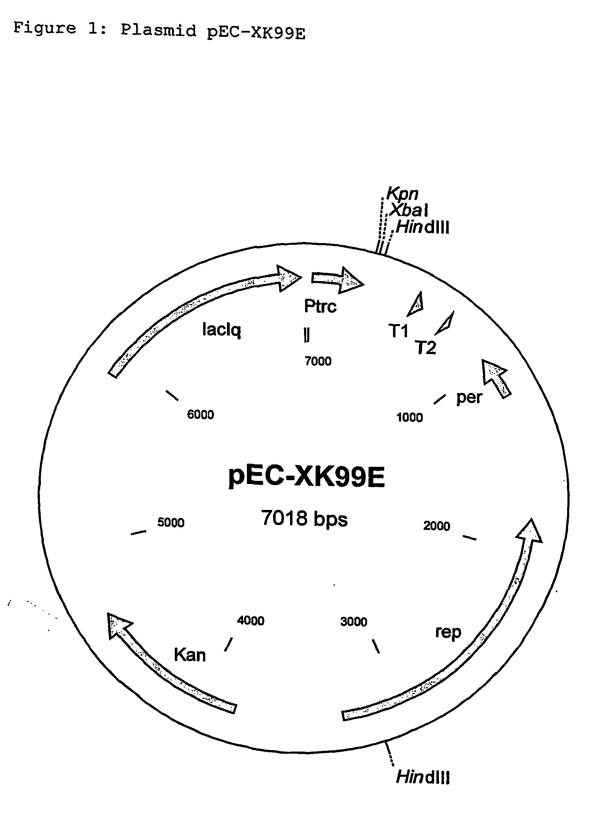

The plasmid construct thus obtained in this way was called pEC-XK99E and is shown in Figure 1. The strain obtained by electroporation of the plasmid pEC-XK99E in the C. glutamicum strain DSM5715 was called DSM5715/pEC-XK99E and deposited as DSM 13455 at the Deutsche Sammlung für Mikroorganismen und Zellkulturen (DSMZ = German Collection of Microorganisms and Cell Cultures, Braunschweig, Germany) in accordance with the Budapest Treaty.

### 3.3 Cloning of the cysD, cysK, cysE and cysH genes in the E. coli-C. glutamicum shuttle vector pEC-XK99E

The E. coli - C. glutamicum shuttle vector pEC-XK99E described in Example 3.1 was used as the vector. DNA of this plasmid was cleaved completely with the restriction enzymes KpnI and XbaI and then dephosphorylated with shrimp alkaline phosphatase (Roche Diagnostics GmbH, Mannheim, Germany, Product Description SAP, Product No. 1758250).

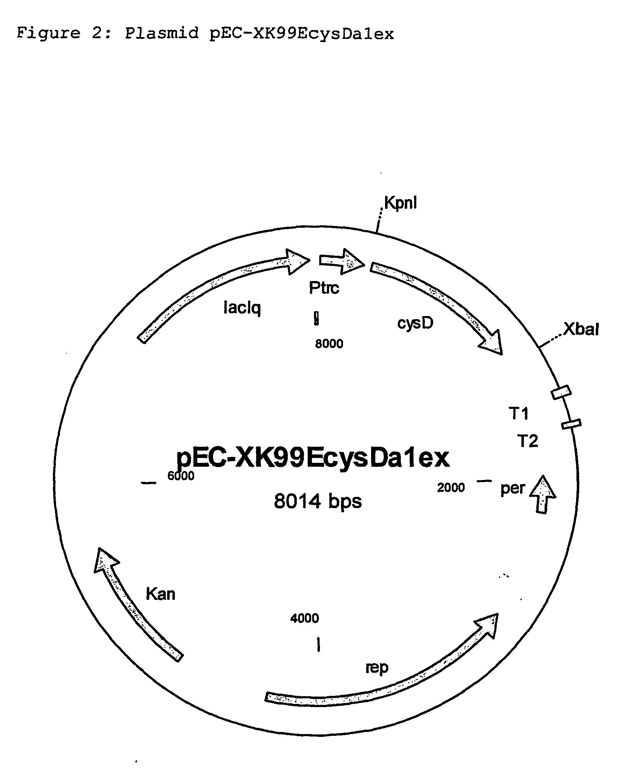

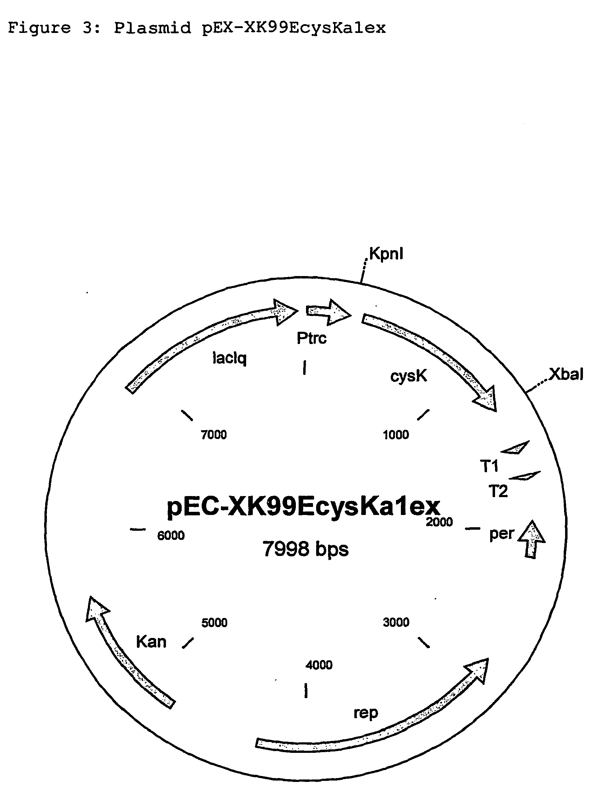

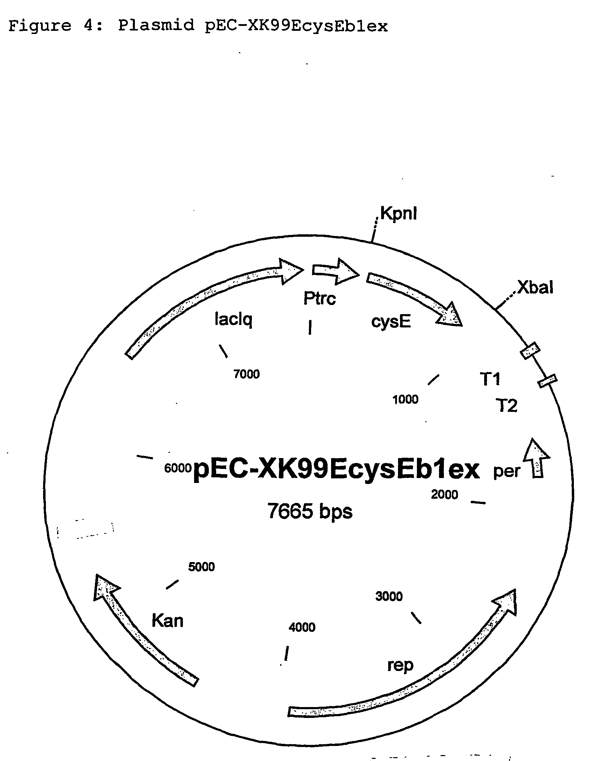

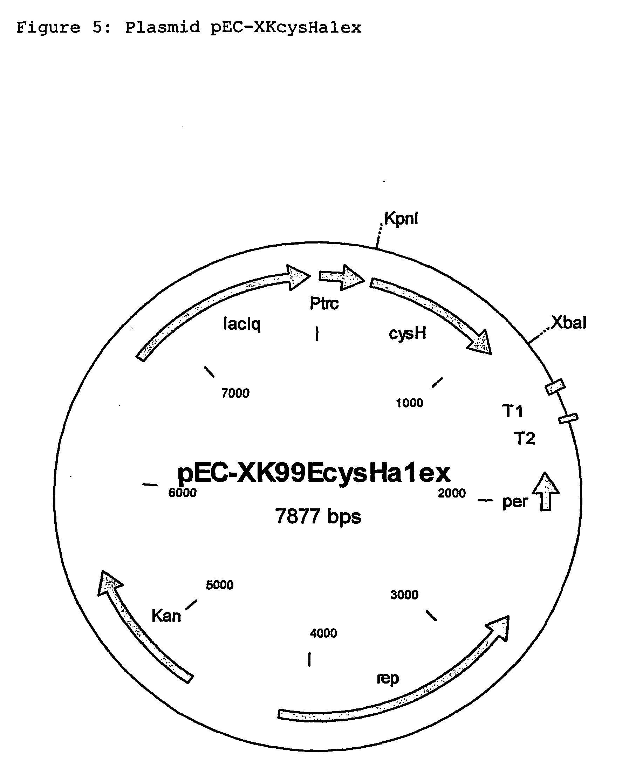

The fragments cysD, approx. 1000 bp in size, cysK, approx. 990 bp in size, cysE, approx. 660 bp in size and cysH, approx. 870 bp in size cleaved with the restriction enzymes KpnI and XbaI and isolated from the agarose gel were in each case mixed with the prepared vector pEC-XK99E and the batches were treated with T4 DNA ligase (Amersham Pharmacia, Freiburg, Germany, Product Description T4-DNA- - Ligase, Code no.27-0870-04). The ligation batches were transformed in the E. coli strain DH5amcr (Hanahan, In: DNA Cloning. A Practical Approach. Vol. I, IRL-Press, Oxford, Washington DC, USA). Selection of plasmid-carrying cells was made by plating out the transformation batches on LB agar (Lennox, 1955, Virology, 1:190) with 50 mg/l kanamycin. After incubation overnight at 37°C, recombinant individual clones were selected. Plasmid DNA was isolated from a transformant in each case with the Qiaprep Spin Miniprep Kit (Product No. 27106, Qiagen, Hilden, Germany) in accordance with the manufacturer's instructions and cleaved with the restriction enzymes KpnI and XbaI to check the plasmid by subsequent agarose gel electrophoresis. The plasmids obtained were called pEC-XK99EcysDa1ex, pEC-XK99EcysKalex, pEC-XK99EcysEblex and pEC-XK99EcysHalex. They are shown in Figures 2, 3, 4 and 5.

### Example 4

### Transformation of the strain DSM5715 with the plasmids pEC-XK99EcysDalex, pEC-XK99EcysKalex, pEC-XK99EcysEblex and pEC-XK99EcysHalex

The strain DSM5715 was transformed with in each case one of the plasmids pEC-XK99EcysDalex, pEC-XK99EcysKalex, pEC-XK99EcysEblex and pEC-XK99EcysHalex using the electroporation method described by Liebl et al., (FEMS Microbiology Letters, 53:299-303 (1989)). Selection of the transformants took place on LBHIS agar comprising 18.5 g/l brain-heart infusion broth, 0.5 M sorbitol, 5 g/l Bactotryptone, 2.5 g/l Bacto-yeast extract, 5 g/l NaCl and 18 g/l Bacto-agar, which had been supplemented with 25 mg/l kanamycin. Incubation was carried out for 2 days at 33°C.

Plasmid DNA was isolated from a transformant in each case by conventional methods (Peters-Wendisch et al., 1998, Microbiology 144, 915-927). DNA of the plasmids pEC-XK99EcysDa1ex, pEC-XK99EcysKalex, pEC-XK99EcysEb1ex and pEC-XK99EcysHa1ex were cleaved with the restriction endonucleases KpnI and XbaI. The plasmids were checked by subsequent agarose gel electrophoresis. The strains obtained were called DSM5715/pEC-XK99EcysDalex, DSM5715/pEC-XK99EcysKalex, DSM5715/pEC-XK99EcysEblex or DSM5715/pEC-XK99EcysHalex.

### Example 5

### Preparation of Lysine

The C. glutamicum strains DSM5715/pEC-XK99EcysDalex, DSM5715/pEC-XK99EcysKalex, DSM5715/pEC-XK99EcysEblex or DSM5715/pEC-XK99EcysHalex obtained in Example 4 were cultured in a nutrient medium suitable for the production of lysine and the lysine content in the culture supernatant of each strain was determined.

For this, the strains were first incubated on an agar plate with the corresponding antibiotic (brain-heart agar with kanamycin (25 mg/l)) for 24 hours at 33°C. Starting from this agar plate culture, in each case a preculture was seeded (10 ml medium in a 100 ml conical flask). The complete medium CgIII was used as the medium for the precultures.

| Medium Cg III | |
|---|---|
| NaCl | 2.5 g/l |
| Bacto-Peptone | 10 g/l |
| Bacto-Yeast extract | 10 g/l |
| Glucose (autoclaved separately) | 2% (w/v) |

The pH was brought to pH 7.4

Kanamycin (25 mg/l) was added to this. The precultures were incubated for 16 hours at 33°C at 240 rpm on a shaking machine. In each case a main culture was seeded from these precultures such that the initial OD (660nm) of the main cultures was 0.1. Medium MM was used for the main cultures.

| Medium MM | |
|---|---|
| CSL (corn steep liquor) | 5 g/l |
| MOPS (morpholinopropanesulfonic acid) | 20 g/l |
| Glucose (autoclaved separately) | 50 g/l |
| (NH₄)₂SO₄ | 25 g/l |
| KH₂PO₄ | 0.1 g/l |
| MgSO₄ * 7 H₂O | 1.0 g/l |
| CaCl₂ * 2 H₂O | 10 mg/l |
| FeSO₄ * 7 H₂O | 10 mg/l |
| MnSO₄ * H₂O | 5.0mg/l |
| Biotin (sterile-filtered) | 0.3 mg/l |
| Thiamine * HCl (sterile-filtered) | 0.2 mg/l |
| L-Leucine (sterile-filtered) | 0.1 g/l |
| CaCO₃ | 25 g/l |

The CSL, MOPS and the salt solution were brought to pH 7 with aqueous ammonia and autoclaved. The sterile substrate and vitamin solutions were then added, as well as the CaCO₃ autoclaved in the dry state.

Culturing was carried out in a 10 ml volume in a 100 ml conical flask with baffles. Kanamycin (25mg/l) was added. Culturing was carried out at 33°C and 80% atmospheric humidity.

After 48 hours the OD of the cultures DSM5715, DSM5715/pEC-XK99EcysDalex, DSM5715/pEC-XK99EcysKalex and DSM5715/pEC-XK99EcysHalex and after 72.hours the OD of the culture DSM5715/pEC-XK99EcysEblex was determined at a measurement wavelength of 660 nm with a Biomek 1000 (Beckmann Instruments GmbH, Munich). The amount of lysine formed was in each case determined with an amino acid analyzer from Eppendorf-BioTronik (Hamburg, Germany) by ion exchange chromatography and post-column derivation with ninhydrin detection.

The result of the experiment is shown in Tables 2 and 3.

**Table 2**

| Strain | OD (660 nm) (48 h) | Lysine HC1 g/l (48 h) |
|---|---|---|
| DSM5715 | 11.3 | 13.11 |
| DSM5715/pEC-XK99EcysDalex | 13.7 | 13.54 |
| DSM5715/pEC-XK99EcysKalex | 13.5 | 14.35 |
| DSM5715/pEC-XK99EcysHalex | 11.5 | 15.22 |

**Table 3**

| Strain | OD (660 nm) (72 h) | Lysine HCl (72 h) g/l |
|---|---|---|
| DSM5715 | 7.17 | 14.27 |
| DSM5715/pEC-XK99EcysEb1ex | 9.0 | 15.22 |

### Brief Description of the Figures:

Figure 1: Map of the plasmid pEC-XK99E
Figure 2: Map of the plasmid pEC-XK99EcysDa1ex
Figure 3: Map of the plasmid pEC-XK99EcysKa1ex
Figure 4: Map of the plasmid pEC-XK99EcysEb1ex
Figure 5: Map of the plasmid pEC-XK99EcysHa1ex

The abbreviations and designations used have the following meaning:
- Kan:: Kanamycin resistance gene aph(3')-IIa from Escherichia coli
- HindIII: Cleavage site of the restriction enzyme HindIII
- XbaI: Cleavage site of the restriction enzyme XbaI
- KpnI: Cleavage site of the restriction enzyme KpnI
- Ptrc ;: trc promoter
- T1: Termination region T1
- T2: Termination region T2
- per: Replication effector per
- rep: Replication region rep of the plasmid pGA1
- lacIq: lacIq repressor of the lac operon of Escherichia coli
- cysD: Cloned cysD gene
- cysK: Cloned cysK gene
- cysE: Cloned cysE gene
- cysH: Cloned cysH gene

## Claims

1. A process for the fermentative preparation of L-lysine, which comprises carrying out the following steps:
a) fermentation of coryneform bacteria which produce L-lysine and in which one or more of the:genes selected from the group consisting of the cysD gene, the cysN gene, the cysK gene, the cysE gene and the cysH gene is recombinantly over-expressed;
b) concentration of the L-lysine in the medium or in the cells of the bacteria, and
c) isolation of the L-lysine,
wherein said cysD gene comprises a polynucleotide which codes for a polypeptide having the activity of a subunit II of sulfate adenylyl transferase and which comprises an amino acid sequence which is 90% identical to the amino acid sequence of SEQ ID No. 2, and
wherein said cysN gene comprises a polynucleotide which codes for a polypeptide having the activity of a subunit I of sulfate adenylyl transferase and which comprises an amino acid sequence which is 90% identical to the amino acid sequence of SEQ ID No. 3, and
wherein said cysK gene comprises a polynucleotide which codes for a polypeptide having the activity of a cysteine synthase A and which comprises an amino acid sequence which is 90% identical to the amino acid sequence of SEQ ID No. 5, and
wherein said cysE gene comprises a polynucleotide which codes for a polypeptide having the activity of a serine acetyl transferase and which comprises an amino acid sequence which is 90% identical to the amino acid sequence of SEQ ID No. 6, and
wherein said cysH gene comprises a polynucleotide which codes for a polypeptide having the activity of a 3'-phosphoadenylyl sulfate reductase and which comprises an amino acid sequence which is 90% identical to the amino acid sequence of SEQ ID No. 8.

2. The process of claim 1,
wherein said subunit II of sulfate adenylyl transferase encoded by said cysD gene comprises the amino acid sequence of SEQ ID No. 2,
wherein said subunit I of sulfate adenylyl transferase encoded by said cysN gene comprises the amino acid sequence of SEQ ID No. 3,
wherein said cysteine synthase A encoded by said cysK gene comprises the amino acid sequence of SEQ ID No. 5,
wherein said serine acetyl transferase encoded by said cysE gene comprises the amino acid sequence of SEQ ID No. 6, and
wherein said 3'-phosphoadenylyl sulfate reductase encoded by said cysH gene comprises the amino acid sequence of SEQ ID No. 8.

3. The process of claim 2,
wherein said cysD gene comprises the nucleotide sequence from position 232 to 1143 of SEQ ID No. 1,
wherein said cysN gene comprises the nucleotide sequence from position 1146 to 2444 of SEQ ID No. 1,
wherein said cysK gene comprises the nucleotide sequence from position 271 to 1203 of SEQ ID No. 4,
wherein said cysE gene comprises the nucleotide sequence from position 1392 to 1955 of SEQ ID No. 4,
wherein said cysH gene comprises the nucleotide sequence from position 250 to 1032 of SEQ ID No.7.

4. The process of any of claims 1 to 3, wherein said coryneform bacterium is of the genus Corynebacterium.

5. The process of claim 4, wherein said Corynebacterium is of the species Corynebacterium glutamicum.

6. The process of any of claims 1 to 5, wherein said overexpxession is achieved by increasing the number of copies of the gene or genes or by using a potent promoter.

7. The process of claim 6, wherein said increasing of the copy number is achieved by integration of at least a second copy into the chromosome.

8. The process of claim 6 or 7, wherein said increasing of the copy number is achieved by using a vector.

9. The process of claim 8, wherein said vector is selected from the group consisting of
a) pEC-XK99EcysEblex deposited as DH5amcr/pEC-XK99EcysEblex under accession number DSM14308,
b) pEC-XK99EcysKalex deposited as DH5amcr/pEC-XK99EcysKalex under accession number DSM 14310,
c) pEC-XK99EcysDalex deposited as DH5amcr/pEC-XK99EcysDalex under accession number DSM 14311, and
d) pEC-XK99EcysHalex deposited as DHSamcr/pEC-XK99EcysHalex under accession number DSM 14315.

10. The process of any of claims 1 to 9, wherein one or more of the genes chosen from the group consisting of
a) the dapA gene which codes for dihydrodipicolinate synthase,
b) the gap gene which codes for glyceraldehyde 3-phosphate dehydrogenase,
c) the tpi gene which codes for triose phosphate isomerase,
d) the pgk gene which codes for 3-phosphoglycerate kinase,
e) the zwf gene which codes for glucose 6-phosphate dehydrogenase,
f) the pyc gene which codes for pyruvate carboxylase,
g) the mqo gene which codes for malate-quinone oxidoreductase,
h) the lysC gene which codes for a feed-back resistant aspartate kinase,
i) the lysE gene which codes for lysine export, and
j) the zwa1 gene which codes for the Zwa1 protein is or are recombinantly over-expressed.

## Patentansprüche

1. Verfahren zur fermentativen Herstellung von L-Lysin, bei dem man folgende Schritte durchführt:
a) Fermentation von L-Lysin produzierenden coryneformen Bakterien, in denen man eines oder mehrere der Gene, ausgewählt aus der Gruppe, bestehend aus dem cysD-Gen, dem cysN-Gen, dem cysK-Gen, dem cysE-Gen und dem cysH-Gen, rekombinant überexprimiert,
b) Anreicherung des L-Lysins im Medium oder in den Zellen der Bakterien und
c) Isolierung des L-Lysins,
wobei das cysD-Gen ein Polynukleotid enthält, das für ein die Aktivität einer Untereinheit II der Sulfat-Adenylyltransferase aufweisendes Polypeptid kodiert und das eine Aminosäuresequenz enthält, die 90% identisch ist mit der Aminosäuresequenz von SEQ ID No. 2, und
wobei das cysN-Gen ein Polynukleotid enthält, das für ein die Aktivität einer Untereinheit I der Sulfat-Adenylyltransferase aufweisendes Polypeptid kodiert und das eine Aminosäuresequenz enthält, die 90% identisch ist mit der Aminosäuresequenz von SEQ ID No. 3, und
wobei das cysK-Gen ein Polynukleotid enthält, das für ein die Aktivität einer Cysteinsynthase A aufweisendes Polypeptid kodiert und das eine Aminosäuresequenz enthält, die 90% identisch ist mit der Aminosäuresequenz von SEQ ID No. 5, und
wobei das cysE-Gen ein Polynukleotid enthält, das für ein die Aktivität einer Serinacetyltransferase aufweisendes Polypeptid kodiert und das eine Aminosäuresequenz enthält, die 90% identisch ist mit der Aminosäuresequenz von SEQ ID No. 6, und
wobei das cysH-Gen ein Polynukleotid enthält, das für ein die Aktivität einer 3'-Phosphoadenylylsulfat-Reduktase aufweisendes Polypeptid kodiert und das eine Aminosäuresequenz enthält, die 90% identisch ist mit der Aminosäuresequenz von SEQ ID No. 8.

2. Verfahren nach Anspruch 1,
wobei die durch das cysD-Gen kodierte Untereinheit II der Sulfat-Adenylyltransferase die Aminosäuresequenz von SEQ ID No. 2 enthält,
wobei die durch das cysN-Gen kodierte Untereinheit I der Sulfat-Adenylyltransferase die Aminosäuresequenz von SEQ ID No. 3 enthält,
wobei die durch das cysK-Gen kodierte Cysteinsynthase A die Aminosäuresequenz von SEQ ID No. 5 enthält,
wobei die durch das cysE-Gen kodierte Serinacetyltransferase die Aminosäuresequenz von SEQ ID No. 6 enthält und
wobei die durch das cysH-Gen kodierte 3'-Phosphoadenylylsulfatreduktase die Aminosäuresequenz von SEQ ID No. 8 enthält.

3. Verfahren nach Anspruch 2,
wobei das cysD-Gen die Nukleotidsequenz der Position 232 bis 1143 von SEQ ID No. 1 enthält,
wobei das cysN-Gen die Nukleotidsequenz der Position 1146 bis 2444 von SEQ ID No. 1 enthält,
wobei das cysK-Gen die Nukleotidsequenz der Position 271 bis 1203 von SEQ ID No. 4 enthält,
wobei das cysE-Gen die Nukleotidsequenz der Position 1392 bis 1955 von SEQ ID No. 4 enthält,
wobei das cysH-Gen die Nukleotidsequenz der Position 250 bis 1032 von SEQ ID No. 7 enthält.

4. Verfahren nach einem der Ansprüche 1 bis 3, bei dem das coryneforme Bakterium zur Gattung Corynebacterium gehört.

5. Verfahren nach Anspruch 4, bei dem das Corynebacterium zur Art Corynebacterium glutamicum gehört.

6. Verfahren nach einem der Ansprüche 1 bis 5, bei dem man zur Überexprimierung die Kopienzahl des Gens oder der Gene erhöht oder einen starken Promotor verwendet.

7. Verfahren nach Anspruch 6, bei dem man zur Erhöhung der Kopienzahl wenigstens eine zweite Kopie in das Chromosom integriert.

8. Verfahren nach Anspruch 6 oder 7, bei dem man zur Erhöhung der Kopienzahl einen Vektor verwendet.

9. Verfahren nach Anspruch 8, bei dem man den Vektor auswählt aus der Gruppe
a) pEC-XK99EcysEb1ex hinterlegt als DH5amcr/pEC-XK99EcysEb1ex unter der Zugangsnummer DSM 14308,
b) pEC-XK99EcysKa1ex hinterlegt als DH5amcr/pEC-XK99EcysKa1ex unter der Zugangsnummer DSM 14310,
c) pEC-XK99EcysDa1ex hinterlegt als DH5amcr/pEC-XK99EcysDa1ex unter der Zugangsnummer DSM 14311,
d) pEC-XK99EcysHalex hinterlegt als DH5amcr/pEC-XK99EcysHalex unter der Zugangsnummer DSM 14315.

10. Verfahren nach einem der Ansprüche 1 bis 9, bei dem man eines oder mehrere der Gene, ausgewählt aus der Gruppe
a) das für Dihydrodipicolinatsynthase kodierende Gen dapA,
b) das für die Glyceraldehyd 3-Phosphatdehydrogenase kodierende Gen gap,
c) das für die Triosephosphatisomerase kodierende Gen tpi,
d) das für die 3-Phosphoglyceratkinase kodierende Gen pgk,
e) das für die Glucose 6-Phosphatdehydrogenase kodierende Gen zwf,
f) das für die Pyruvatcarboxylase kodierende Gen pyc,
g) das für die Malat-Chinon-Oxidoreduktase kodierende Gen mqo,
h) das für eine feed back resistente Aspartatkinase kodierende Gen lysC,
i) das für den Lysinexport kodierende Gen lysE und
j) das für das Zwal-Protein kodierende Gen zwa1 rekombinant überexprimiert.

## Revendications

1. Procédé de préparation de L-lysine par fermentation, qui comprend la réalisation des étapes suivantes :
a) la fermentation de bactéries coryneformes qui produisent de la L-lysine et dans lesquelles un ou plusieurs des gènes choisis dans le groupe constitué du gène cysD, du gène cysN, du gène cysK, du gène cysE et du gène cysH, sont surexprimés sous forme recombinante ;
b) la concentration de la L-lysine dans le milieu ou dans les cellules des bactéries, et
c) l'isolement de la L-lysine,
dans lequel ledit gène cysD comprend un polynucléotide qui code pour un polypeptide exerçant l'activité d'une sous-unité II de sulfate-adénylyle transférase et qui comprend une séquence d'acides aminés qui est identique à 90 % à la séquence d'acides aminés représentée par la SEQ ID n° 2, et
dans lequel ledit gène cysN comprend un polynucléotide qui code pour un polypeptide exerçant l'activité d'une sous-unité I de sulfate-adénylyle transférase et qui comprend une séquence d'acides aminés qui est identique à 90 % à la séquence d'acides aminés représentée par la SEQ ID n° 3, et
dans lequel ledit gène cysK comprend un polynucléotide qui code pour un polypeptide exerçant l'activité d'une cystéine synthase A et qui comprend une séquence d'acides aminés qui est identique à 90 % à la séquence d'acides aminés représentée par la SEQ ID n° 5, et
dans lequel ledit gène cysE comprend un polynucléotide qui code pour un polypeptide exerçant l'activité d'une sérine-acétyle transférase et qui comprend une séquence d'acides aminés qui est identique à 90 % à la séquence d'acides aminés représentée par la SEQ ID n° 6, et
dans lequel ledit gène cysH comprend un polynucléotide qui code pour un polypeptide exerçant l'activité d'une 3'-phosphoadénylylsulfate réductase et qui comprend une séquence d'acides aminés qui est identique à 90 % à la séquence d'acides aminés représentée par la SEQ ID n° 8.

2. Procédé selon la revendication 1,
dans lequel ladite sous-unité II de la sulfate-adénylyle transférase codée par ledit gène cysD comprend la séquence d'acides aminés représentée par la SEQ ID n° 2,
dans lequel ladite sous-unité I de la sulfate-adénylyle transférase codée par ledit gène cysN comprend la séquence d'acides aminés représentée par la SEQ ID n° 3,
dans lequel ladite cystéine synthase A codée par ledit gène cysK comprend la séquence d'acides aminés représentée par la SEQ ID n° 5,
dans lequel ladite sérine-acétyle transférase codée par ledit gène cysE comprend la séquence d'acides aminés représentée par la SEQ ID n° 6, et
dans lequel ladite 3'-phosphoadénylylsulfate réductase codée par ledit gène cysH comprend la séquence d'acides aminés représentée par la SEQ ID n° 8.

3. Procédé selon la revendication 2,
dans lequel ledit gène cysD comprend la séquence de nucléotides s'étendant de la position 232 à la position 1143 de la SEQ ID n° 1,
dans lequel ledit gène cysN comprend la séquence de nucléotides s'étendant de la position 1146 à la position 2444 de la SEQ ID n° 1,
dans lequel ledit gène cysK comprend la séquence de nucléotides s'étendant de la position 271 à la position 1203 de la SEQ ID n° 4,
dans lequel ledit gène cysE comprend la séquence de nucléotides s'étendant de la position 1392 à la position 1955 de la SEQ ID n° 4,
dans lequel ledit gène cysH comprend la séquence de nucléotides s'étendant de la position 250 à la position 1032 de la SEQ ID n° 7.

4. Procédé selon l'une quelconque des revendications 1 à 3, dans lequel ladite bactérie coryneforme est du genre Corynebacterium.

5. Procédé selon la revendication 4, dans lequel ladite bactérie Corynebacterium est de l'espèce Corynebacterium glutamicum.

6. Procédé selon l'une quelconque des revendications 1 à 5, dans lequel la surexpression est obtenue en accroissant le nombre de copies du ou des gènes ou en utilisant un promoteur puissant.

7. Procédé selon la revendication 6, dans lequel ledit accroissement du nombre de copies est obtenu par intégration d'au moins une deuxième copie au chromosome.

8. Procédé selon la revendication 6 ou 7, dans lequel ledit accroissement du nombre de copies est obtenu en utilisant un vecteur.

9. Procédé selon la revendication 8, dans lequel ledit vecteur est choisi dans le groupe constitué de :
a) pEC-XK99EcysEb1ex déposé sous la désignation DH5amcr/pEC-XK99EcysEb1ex sous le numéro d'accès DSM14308,
b) pEC-XK99EcysKalex déposé sous la désignation DH5amcr/pEC-XK99EcysKalex sous le numéro d'accès DSM 14310,
c) pEC-XK99EcysDalex déposé sous la désignation DH5amcr/pEC-XK99EcysDalex sous le numéro d'accès DSM 14311, et
d) pEC-XK99EcysHalex déposé sous la désignation DH5amcr/pEC-XK99EcysHalex sous le numéro d'accès DSM 14315.

10. Procédé selon l'une quelconque des revendications 1 à 9, dans lequel un ou plusieurs des gènes choisis dans le groupe constitué :
a) du gène dapA, qui code pour la dihydrodipicolinate synthase,
b) du gène gap, qui code pour la glycéraldéhyde-3-phosphate déshydrogénase,
c) du gène tpi qui code pour la triose-phosphate isomérase,
d) du gène pgk qui code pour la 3-phosphoglycérate kinase,
e) du gène zwf qui code pour la glucose-6-phosphate déshydrogénase,
f) du gène pyc qui code pour la pyruvate carboxylase,
g) du gène mqo qui code pour la malate-quinone oxydoréductase,
h) du gène lysC qui code pour une aspartate kinase résistant à la rétroaction,
i) du gène lysE qui code pour une protéine d'export de la lysine, et
j) du gène zwa1 qui code pour la protéine Zwa1, est ou sont surexprimés sous forme recombinante.
